# EUROPEAN PATENT SPECIFICATION

(11) **EP 4 262 946 B1**
(45) Date of publication and mention of the grant of the patent: **01.07.2026**
(21) Application number: 21907913.4
(22) Date of filing: 17.12.2021
(51) Int. Cl.: A61M 16/04, A61M 25/01, A61B 1/267

(54) **FLEXIBLE ARTICULATING INTUBATION TOOL**
FLEXIBLES GELENKIGES INTUBATIONSWERKZEUG
OUTIL D'INTUBATION FLEXIBLE ARTICULÉ

(30) Priority: 18.12.2020 US 202063127986 P; 01.03.2021 US 202163155301 P
(43) Date of publication of application: 25.10.2023
(73) Proprietor: TJB Medical, Inc., Columbus, MN 55025 (US)
(72) Inventor: HORRISBERGER, Benn Douglas, Blaine, Minnesota 55449 (US); MOFFAT, Thomas Brian, Hugo, Minnesota 55038 (US); MELANDER, David, St. Louis Park, Minnesota 55416 (US)
(74) Representative: Potter Clarkson
(86) International application number: PCT/US2021/064089
(87) International publication number: WO 2022/133248

(56) References cited:
- EP-A1- 3 335 755
- WO-A1-2018/202720
- US-A1- 2008 058 599
- US-A1- 2012 078 050
- US-A1- 2014 238 390
- US-A1- 2018 110 950
- US-A1- 2018 110 950
- US-A1- 2020 254 204

## Description

### Field

The present application is directed to a tool for aiding in endotracheal intubation, in particular a tool for assisting in placement of an endotracheal tube into a trachea.

### Background

Approximately 8% of the population has partial to zero visual laryngeal exposure resulting in difficult intubation. In many instances, this partial view caused by anatomical variances inhibits placement of an endotracheal tube into the trachea. This inhibition is often due to the inability to manipulate the end of the rigid stylet and (superimposed endotracheal tube) while performing intubation. The spatial limitations imposed allow only modest movements of the stylet/endotracheal tube. Anatomical variances further inhibit the visualization of the trachea mandating precise control of the stylet/ endotracheal tube for successful intubation of the trachea. Therefore, a need exists for a means to aid in placement of an endotracheal tube into a trachea.

Patent document US2018/110950A1 and US2014/238390A1 are hereby acknowledged.

### Summary of the Invention

The invention is defined by the appended claims.

The present disclosure is directed to an intubation tool for assisting in placement of an endotracheal tube. The intubation tool comprises a stylet having a proximal end and a distal end, the stylet configured for receiving an endotracheal tube. The stylet has an articulating distal end, the articulating distal end being configured to articulate between more than one shape. The tool also includes a handle secured to the proximal end of the stylet.

In an embodiment, an intubation tool for assisting in placement of an endotracheal tube, the intubation tool is included having a) a stylet having a proximal end, a distal end, and a medial location, the stylet: i) configured for receiving an endotracheal tube, and ii) having an articulating distal tip, the articulating distal tip being configured to articulate between more than one shape, and b) a removable handle secured to a medial location of the stylet.

In an embodiment, articulation of the distal tip of the stylet can be accomplished with a single hand that holds the removable handle and articulates the distal end.

In an embodiment, articulation of the distal tip of the stylet can be accomplished with a single finger manipulating a steering control mounted on the stylet.

In an embodiment, articulation of the distal tip of the stylet can be accomplished with two fingers manipulating a steering control mounted on the stylet, the steering control sliding along the stylet.

In an embodiment, articulation of the distal end of the stylet can be accomplished with three fingers from a single hand that articulates the distal end.

In an embodiment, the articulating distal tip can be articulated with a force of less than 9 Newton. Generally, the force necessary move the distal tip should be relatively small so that just the simple force applied by a few figures (such as one, two, or three fingers) is adequate to manipulate the distal tip of the stylet. Note that just one, two or three fingers can be used to easily articulate the tip from a neutral position to a positive or negative position without ever releasing grip on the handle. Thus, there is a continuous range of movement from positive to neutral to distal positions (and intermediate positions), all within the fingers in contact with the tool the whole time and make the movement of the tip. This allows for very precise manipulation of the tip, but also allows for sensitive feedback to the operator's fingers. In this way the operator is able to get multiple types of feedback: They can get the feedback of resistance to articulation (and thus sensing where they are within a patient's anatomy), but also get feedback from the tip while advancing it into the patient. **In** this manner the operator has unparalleled awareness to the patient's anatomy and the position of the tip as it moves through the anatomy.

In an embodiment, the articulating distal tip can be articulated with a force of from 4 to 9 Newton.

In an embodiment, the articulating distal tip is deformable upon contact with an obstruction.

In an embodiment, the articulating distal tip transmits force back to steering control upon contact with an obstruction.

In an embodiment, translation movement of the steering control results in bending articulation of the articulating distal tip.

In an embodiment, the translation movement of the steering control by a distance D results in bending articulation of the distal tip by a distance of at least 150 percent of D. As used herein, D refers to the distance the steering control is moved along the axis of the stylet, either toward or away from the distal tip. For example, the distance D could be 1 centimeter, in which case 150 percent deflection of the distal tip would be 1.5 centimeters. In alternative embodiments the articulation of the distal tip can be less than 100 percent or more than 150 percent. Also, it will be appreciated that in some embodiments there is a non-linear relationship between translational movement of the steering control and bending articulation of the distal tip. Thus, the amount of translational movement can result in either increased or decreased bending (deflection) of the tip at different points along the path of travel of the steering control.

In an embodiment, translational movement of the steering control in a first direction results in bending articulation of the articulating distal tip in a first direction, and translational movement of the steering control in an opposite direction results in bending articulation of the articulating distal tip in a direction opposite the first direction.

In an embodiment, the stylet includes tubing containing at least one lumen.

In an embodiment, the stylet includes bilumen tubing.

In an embodiment, upon removal of the handle from the medial portion of the stylet, the medial portion of the stylet has a diameter no greater than 120 percent of the remainder of the stylet.

In an embodiment, the widest portion of the stylet after removal of the handle is less than the diameter of the interior of the lumen of an endotracheal tube to be installed on the stylet.

In an embodiment, the handle and stylet provide a clear sight line of angle α of 20 degrees along a dorsal elevation L_{D} of 1 cm.

In an embodiment, the handle and stylet provide a clear sight line of angle α of 20 degrees along a dorsal elevation L_{D} of 2 cm.

In an embodiment, the handle and stylet provide a clear sight line of angle α of 20 degrees along a dorsal elevation L_{D} of 3 cm.

In an embodiment, the handle and stylet provide a clear sight line of angle α of 90 degrees along a dorsal elevation L_{D} of 1 cm.

In an embodiment, the handle and stylet provide a clear sight line of angle α of 180 degrees along a dorsal elevation L_{D} of 3 cm.

In an embodiment, the handle and stylet provide a clear sight line of angle α of 270 degrees along a dorsal elevation L_{D} of 3 cm.

In an embodiment, the stylet has a length at least 1.5 times the length of an endotracheal tube to be installed on the stylet.

In an embodiment, the stylet has a length at least 2.0 times the length of an endotracheal tube to be installed on the stylet.

In an embodiment, wherein handle and stylet provide a substantially clear sight-line along the length of the stylet when the stylet is in a non-articulated mode.

In an embodiment, wherein handle and stylet provide a substantially clear sight line of at least 180 degrees along the dorsal line of the handle and stylet combination.

In an embodiment, wherein handle and stylet provide a substantially clear sight line of at least 90 degrees along a dorsal line of the handle and stylet combination, within 1 cm of the central axis of the stylet.

In an embodiment, wherein handle and stylet provide a substantially clear sight line of at least 90 degrees along a dorsal line of the handle and stylet combination, within 2 cm of the central axis of the stylet.

In an embodiment, wherein handle and stylet provide a substantially clear sight line of at least 270 degrees along a dorsal line of the handle and stylet combination, within 1 cm of the central axis of the stylet.

In an embodiment, the handle is removable from the medial location of the stylet to allow placement of an endotracheal tube.

In an embodiment, the stylet articulates in a plane.

In an embodiment, the plane in which the stylet articulates can be selected by rotation of the handle, resulting in rotation of the stylet.

In an embodiment, the stylet includes an internal mechanism for articulating the distal end.

In an embodiment, the stylet includes an internal cable connected to the distal end, and wherein pulling on the cable from the medial location results in articulating the distal end of the stylet.

In an embodiment, a method for placement of an endotracheal tube, the method is included, the method a) providing an intubation tool for assisting in placement of an endotracheal tube, the intubation tool is included, the method i) a stylet having a proximal end, distal end, and intermediate medial portion, the stylet configured for receiving an endotracheal tube, and having an articulating distal tip, the articulating distal tip being configured to articulate between more than one shape using one to three fingers, and ii) a handle secured to a medial position on the stylet, b) inserting the distal end of the stylet of the intubation tool into the trachea of a person, including articulating the distal end of the stylet during insertion to aid in passage through the larynx, c) removing the handle from the medial portion of the stylet, d) placing an endotracheal tube over the medial location of the stylet and down toward the distal end of the stylet and into the trachea of the person, and e) removing the stylet from the patient's trachea while keeping the endotracheal tube in place.

In an embodiment, the stylet articulates in a plane.

In an embodiment, the stylet includes an internal mechanism for articulating the distal tip.

In an embodiment, the stylet further includes a flexible intermediate portion.

In an embodiment, the stylet includes an internal cable connected to the distal tip, and wherein pulling on the cable from the medial location results in articulating the distal tip of the stylet.

In an embodiment, further can include an actuator.

In an embodiment, the actuator includes a tube surrounding a portion of the medial location of the stylet.

In an embodiment, wherein handle and stylet provide a substantially clear sight-line along the length of the stylet when the stylet is in a non-articulated mode.

In an embodiment, wherein handle and stylet provide a substantially clear sight line of at least 180 degrees along the dorsal line of the handle and stylet combination.

In an embodiment, wherein handle and stylet provide a substantially clear sight line of at least 90 degrees along a dorsal line of the handle and stylet combination, within 1 cm of the central axis of the stylet.

In an embodiment, wherein handle and stylet provide a substantially clear sight line of at least 90 degrees along a dorsal line of the handle and stylet combination, within 2 cm of the central axis of the stylet.

In an embodiment, wherein handle and stylet provide a substantially clear sight line of at least 270 degrees along a dorsal line of the handle and stylet combination, within 1 cm of the central axis of the stylet.

In an embodiment, an intubation tool for assisting in placement of an endotracheal tube, the endotracheal intubation tool is included having a) a stylet having a medial location and a distal end, the stylet: i.) configured for receiving an endotracheal tube, and ii) having an articulating distal tip, the articulating tip end being configured to articulate between more than one shape, and b) a handle secured to the medial location of the stylet, wherein actuation is provided by a cable.

In an embodiment, the cable radial location optimized to facilitate articulation and minimize pull force.

In an embodiment, the handle is removable from the stylet.

In an embodiment, the handle is removable from the medial location of the stylet to allow placement of an endotracheal tube.

In an embodiment, the stylet articulates in a plane.

In an embodiment, the stylet includes an internal mechanism for articulating the distal end.

In an embodiment, where the stylet further includes a flexible intermediate portion.

In an embodiment, the stylet includes an internal cable connected to the distal end, and wherein pulling on the cable from the medial location results in articulating the distal end of the stylet.

In an embodiment, further can include an actuator.

In an embodiment, the actuator includes a tube surrounding a portion of the medial location of the stylet.

In an embodiment, a method for placement of an endotracheal tube, the method is included, the method a) providing an intubation tool for assisting in placement of an endotracheal tube, the intubation tool is included, the method i) a stylet having a medial location and a distal end, the stylet configured for receiving an endotracheal tube, and having an articulating distal end, the articulating distal end being configured to articulate between more than one shape, and ii) a handle secured to the medial location of the stylet, b) inserting the distal end of the stylet of the endotracheal intubation tool into the trachea of a person, including articulating the distal end of the stylet during insertion to aid in passage through the larynx, c) removing the handle from the medial location of the stylet, d) inserting an endotracheal tube over the medial location of the stylet and down toward the distal end of the stylet, and e) removing the stylet from the patient's trachea while keeping the endotracheal tube in place, wherein the actuation can be performed with a single hand.

In an embodiment, the endotracheal intubation tool for assisting in placement of an endotracheal tube is removable from the medial location of the stylet.

In an embodiment, wherein actuation occurs push/pull action on the tip by allowing 3 finger, 360 degree contact of circular trigger.

In an embodiment, the endotracheal intubation stylet includes an internal mechanism for articulating the distal end.

In an embodiment, the stylet further includes a flexible intermediate portion.

In an embodiment, the stylet includes an internal cable connected to the distal end, and wherein pulling on the cable from the medial location results in articulating the distal end of the stylet.

In an embodiment, further can include an actuator.

In an embodiment, the actuator includes a tube surrounding a portion of the medial location of the stylet.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims .

### Brief Description of the Figures

Aspects may be more completely understood in connection with the following drawings, in which:
FIG. 1 is a drawing showing a side plan view of endotracheal intubation tool for assisting in placement of an endotracheal tube made in accordance with an example embodiment.
FIG. 2 is a drawing showing the position of a patient's head and neck in advance of insertion of an endotracheal tube, showing the approximate oral axis, pharyngeal axis and laryngeal axis.
FIG. 3 is a drawing showing a patient with an endotracheal intubation tool for assisting in placement of an endotracheal tube during initial insertion of the distal end of the endotracheal intubation tool through the patient's mouth.
FIG. 4 is a drawing showing a patient with an endotracheal intubation tool for assisting in placement of an endotracheal tube during initial insertion of the distal end of the endotracheal intubation tool through the patient's mouth, the tool substantially inserted into the patient.
FIG. 5 is a drawing showing a patient with an endotracheal intubation tool for assisting in placement of an endotracheal tube during initial insertion of the distal end of the endotracheal intubation tool through the patient's mouth, the tool substantially inserted into the patient and with the handle removed.
FIG. 6 is a drawing showing a patient with a endotracheal intubation tool for assisting in placement of an endotracheal tube during initial insertion of the distal end of the endotracheal intubation tool through the patient's mouth, the tool substantially inserted into the patient and with the handle removed and the endotracheal tube positioned over the tool stylet and into the patient.
FIG. 7 is a drawing showing a side plan view of endotracheal intubation tool for assisting in placement of an endotracheal tube made in accordance with an example embodiment, showing the stylet of the tool with a removable handle clipped in place.
FIG. 8 is a drawing showing a side plan view of endotracheal intubation tool for assisting in placement of an endotracheal tube made in accordance with an example embodiment, showing the stylet of the tool with the handle removed.
FIG. 9 is a front view of the distal side of a handle of an endotracheal intubation tool with the stylet of the tool installed.
FIG. 10 is a front view of the distal side of a handle of an endotracheal intubation tool with the stylet of the tool installed and handle catch open.
FIG. 11 is a front view of the distal side of a handle of an intubation tool with the stylet of the tool removed and handle catch open.
FIG. 12 is a diagram showing the field of view from an intubation tool made in accordance with the present disclosure.
FIG. 13 is a diagram showing the field of view from an endotracheal intubation tool made in accordance with the present disclosure.
FIG. 14 is view of the distal end of an intubation tool stylet, showing the tip of the stylet in a neutral position.
FIG. 15 is view of the distal end of an intubation tool stylet, showing the tip of the stylet in a downwardly flexed position.
FIG. 16 is view of the distal end of an intubation tool stylet, showing the tip of the stylet in an upwardly flexed position.
FIG. 17 is a closeup view of the steering base of an intubation tool stylet.
FIG. 18 is view of the distal end of an intubation tool stylet, with the tip of the stylet articulated
FIG. 19 is close up view of the medial portion of a stylet, showing the steering control in a substantially neutral location.
FIG. 20 is close up view of the medial portion of a stylet, showing the steering control in a substantially distal location.
FIG. 21 is close up view of the medial portion of a stylet, showing the steering control in a substantially proximal location.
FIG. 22 is a drawing showing an exploded view of components of an endotracheal intubation tool for assisting in placement of an endotracheal tube made in accordance with an example embodiment.
FIG. 23 is a perspective view of a steering base made in accordance with an example embodiment.
FIG. 24 is a side elevational view of a steering base made in accordance with an example embodiment.
FIG. 25 a distal end view of a steering base made in accordance with an example embodiment.
FIG. 26 is a cross sectional view of a steering base made in accordance with an example embodiment.
FIG. 27 is a perspective view of the proximal end cap of an intubation tool stylet in accordance with an embodiment.
FIG. 28 is a distal end view of the proximal end cap of an intubation tool stylet in accordance with an embodiment.
FIG. 29 is a side view of the proximal end cap of an intubation tool stylet in accordance with an embodiment.
FIG. 30 is a perspective view of steering cable tip of an intubation tool stylet in accordance with an embodiment.
FIG. 31 is a proximal end view of steering cable tip of an intubation tool stylet in accordance with an embodiment.
FIG. 32 is a side view of steering cable tip of an intubation tool stylet in accordance with an embodiment.
FIG. 33 is a perspective view of steering control of an intubation tool stylet in accordance with an embodiment.
FIG. 34 is an end view of steering control of an intubation tool stylet in accordance with an embodiment.
FIG. 35 is a side view of steering control of an intubation tool stylet in accordance with an embodiment, with a partial cross section.
FIG. 36 is a perspective view of an articulation collar of an intubation tool stylet in accordance with an embodiment.
FIG. 37 is a side view of an articulation collar of an intubation tool stylet in accordance with an embodiment.
FIG. 38 is an end view of an articulation collar of an intubation tool stylet in accordance with an embodiment.
FIG. 39 is a drawing showing a side plan view of endotracheal intubation tool for assisting in placement of an endotracheal tube made in accordance with an example embodiment.
FIG. 40 is closeup of the endotracheal intubation tool of FIG. 39, enlarging the portion from dotted circle 39A.
FIG. 41 is a drawing showing an exploded view of components of an endotracheal intubation tool for assisting in placement of an endotracheal tube made in accordance with an example embodiment.
FIG. 42 is a drawing showing a side perspective view of endotracheal intubation tool for assisting in placement of an endotracheal tube made in accordance with an example embodiment, showing the articulating tip in an upward (or dorsal) articulation.
FIG. 43 is a drawing showing a side perspective view of endotracheal intubation tool for assisting in placement of an endotracheal tube made in accordance with an example embodiment, showing the articulating tip in a downward (or ventral) articulation.
FIG. 44 is drawing showing the articulating tip of an endotracheal intubation tool in a neutral position.
FIG. 45 is a perspective view of steering control of an intubation tool stylet in accordance with an embodiment.
FIG. 46 is a side view of steering control of an intubation tool stylet in accordance with an embodiment, with a partial cross section.
FIG. 47 is an end view of steering control of an intubation tool stylet in accordance with an embodiment.
FIG. 48 is a perspective view of a vertebrate from the articulating end of an articulating tip, showing the vertebrate independent of other components.
FIG. 49 is a side view of a vertebrate from the articulating end of an articulating tip, showing the vertebrate independent of other components.
FIG. 50 is an end view of a vertebrate from the articulating end of an articulating tip, showing the vertebrate independent of other components.
FIG. 51 is a perspective view of the tip member from an articulating end of an articulating tip, showing the tip member independent of other components.
FIG. 52 is a side cross-sectional view of the tip member from an articulating end of an articulating tip, showing the tip member independent of other components.
FIG. 53 is an end view of the tip member from an articulating end of an articulating tip, showing the tip member independent of other components.
FIG. 54 is a perspective view of a base member from the proximal end of an articulating tip, showing the base member independent of other components.
FIG. 55 is a side view of a base member from the proximal end of an articulating tip, showing the base member independent of other components.
FIG. 56 is a side elevational view of components of an articulating tip of an endotracheal intubation tool in a neutral position.
FIG. 57 is a side elevational view of components of an articulating tip of an endotracheal intubation tool in a partially articulated position.
FIG. 58 is a side elevational view of components of an articulating tip of an endotracheal intubation tool in a partially articulated position.
FIG. 59 is a side elevational view of components of an articulating tip of an endotracheal intubation tool in a partially articulated position.
FIG. 60 is a side elevational view of components of an articulating tip of an endotracheal intubation tool in a partially articulated position.
FIG. 61 is a side elevational view of components of an articulating tip of an endotracheal intubation tool in a partially articulated position.
FIG. 62 is a side elevational view of components of an articulating tip of an endotracheal intubation tool in a partially articulated position.
FIG. 63 is a side elevational view of components of an articulating tip of an endotracheal intubation tool in a partially articulated position, making contact with an obstruction.
FIG. 64 is a side elevational view of components of an articulating tip of an endotracheal intubation tool in a partially articulated position, making contact with an obstruction.
FIG. 65 is a side view of a handle of an endotracheal intubation tool with the stylet of the tool removed.
FIG. 66 is a perspective view of a clasp of a handle of an endotracheal intubation tool.
FIG. 67 is a perspective view of a clasp of a base of an endotracheal intubation tool.
FIG. 68 is a graph showing force relative to articulation position of an articulating tip of an endotracheal intubation tool.
FIG. 69 is a graph showing force relative to articulation position of an articulating tip of an endotracheal intubation tool.
FIG. 70 is a side cross-sectional of a vertebrate from an articulating end of an articulating tip, showing the vertebrate independent of other components.
FIG. 71 is a graph showing required to articulate a vertebrate relative to flat portion of the vertebrate.

While embodiments are susceptible to various modifications and alternative forms, specifics thereof have been shown by way of example and drawings and will be described in detail. It should be understood, however, that the scope herein is not limited to the particular embodiments described.

### Detailed Description

The present disclosure is directed to a device for aiding in endotracheal intubation, in particular a tool for assisting in placement of an endotracheal tube into a trachea.

The device (also referred to herein as a "tool") includes a) a stylet having a proximal end, a distal end, and a medial location, the stylet: i) configured for receiving an endotracheal tube, and ii) having an articulating distal tip, the articulating distal tip being configured to articulate between more than one shape, and b) a removable handle secured to a medial location on of the stylet. In an embodiment the intubation tool comprises a stylet having a proximal end and a distal end, the stylet configured for receiving an endotracheal tube. The stylet has an articulating distal end, the articulating distal end being configured to articulate between more than one shape. The tool also includes a handle secured to the proximal end of the stylet. Optionally articulation of the distal tip of the stylet can be accomplished with a single hand that holds the removable handle and articulates the distal end; and optionally articulation of the distal tip of the stylet can be accomplished with a single finger or two fingers manipulating a steering control mounted on the stylet.

The device provides a means for a clinician to apply a low axial force to the articulation collar in order to fully articulate the distal end. The means by which this is possible comes from having a low friction mechanism that allows the cable to slidably pass around the proximal transition of the steering base. This mechanism/design can include a pully-type mechanism or a design which optimizes radius and surface finish to provide a low friction surface and pathway. This radius and surface finish chosen will be highly dependent on the materials and construction of the cable itself. In order to provide tactile feedback to the clinician, who is manipulating the medial section of the device, the distal tip typically is in intimate contact with the next most proximal, rigid component, and that component to the next and so on. This contact can be accomplished by pre-tensioning the articulation cable, which places the device in tension from the proximal steering transition to the distal tip of the device. For the benefits of all of these design features to be realized, they must all fit within a narrow profile, such as a 6 mm diameter envelope so as to facilitate the sliding of a standard intubation tube along its length.

The device allows for two-way articulation in a plane. This two-way can be quite important because it allows more precise placement of the stylet of the device while navigating the tip of the stylet through a patient's anatomy. Specifically, for example, a clinician must typically cause the tip of the stylus to first pass under the epiglottis, and then flex steeply upward to move past it, next straighten to advance deeper, then flex downward steeply to get over the subglottis, past the vocal chords, into the trachea. A single directional bend is not sufficient to manage entrance in a difficult airway without disturbing a patient's tissue, often causing discomfort and even potential injury.

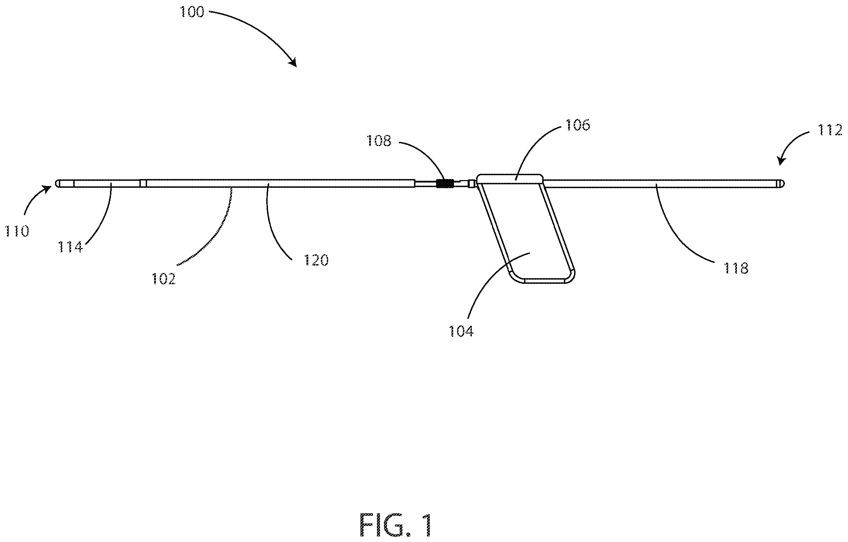

Now in reference to the drawings, FIG. 1 is a drawing showing a side plan view of intubation tool 100 for assisting in placement of an endotracheal tube made in accordance with an example embodiment. The intubation tool 100 includes a stylet 102 having distal end 110 and proximal end 112 that is flexible and has an articulating tip 114 that allows for precise placement during intubation of a patient, thereby allowing placement with minimal disturbance or damage to the patient. The intubation tool 100 further includes a removable handle 104, shown in an example configuration (with other configurations possible). The removable handle 104 can be gripped during an intubation procedure during which the stylus is placed down the trachea of a patient. After the stylus is positioned within the patient the removable handle 104 is removed and an intubation tube slipped over the stylet 102 and into the patient's trachea (the stylet 102 serving as a guide for the intubation tube), after which the stylet 102 is removed while the intubation tube remains extended partially into the trachea of the intubated patient. Removal of the handle 104 is accomplished in the depicted embodiment by opening clasp 106, as shown later herein. The stylet 102 includes a steering control 108 that can slide forward and backward so as to change the shape of articulating tip 114. Stylet 102 further includes a proximal portion 118 and a distal portion 120, both of which are typically flexible but not articulating. Proximal portion 118 of stylet 102 remains outside of a patient during the intubation procedure, while at least some of distal portion 120 is inserted into the patient.

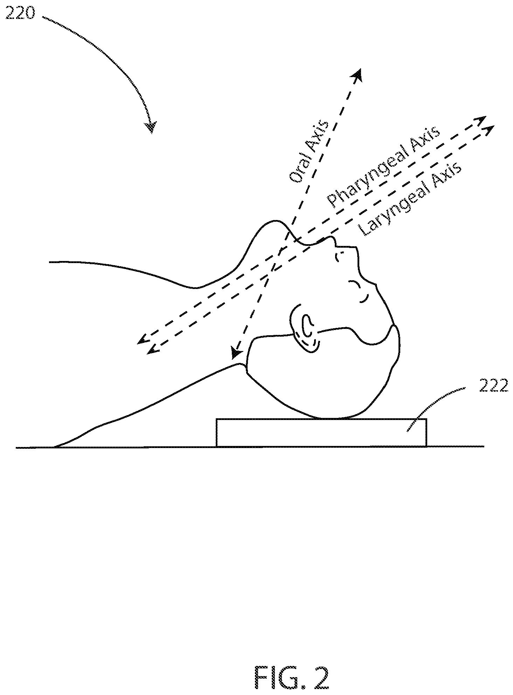

FIG. 2 is a drawing showing the position of the head and neck of a patient 220 in advance of insertion of an endotracheal tube, showing the approximate oral axis, pharyngeal axis and laryngeal axis. The patient's head is shown elevated and tilted by support 222 so as to open up access to the patient's airway. As shown in FIG. 2 the oral axis crosses the pharyngeal axis and laryngeal axis, but is not perfectly in-line, and can change as the head of the patient tilts. This geometry can vary by patient and results in challenges during intubation because the pathway for inserting the intubation tube is often partially obscured and/or can be somewhat curved or non-linear.

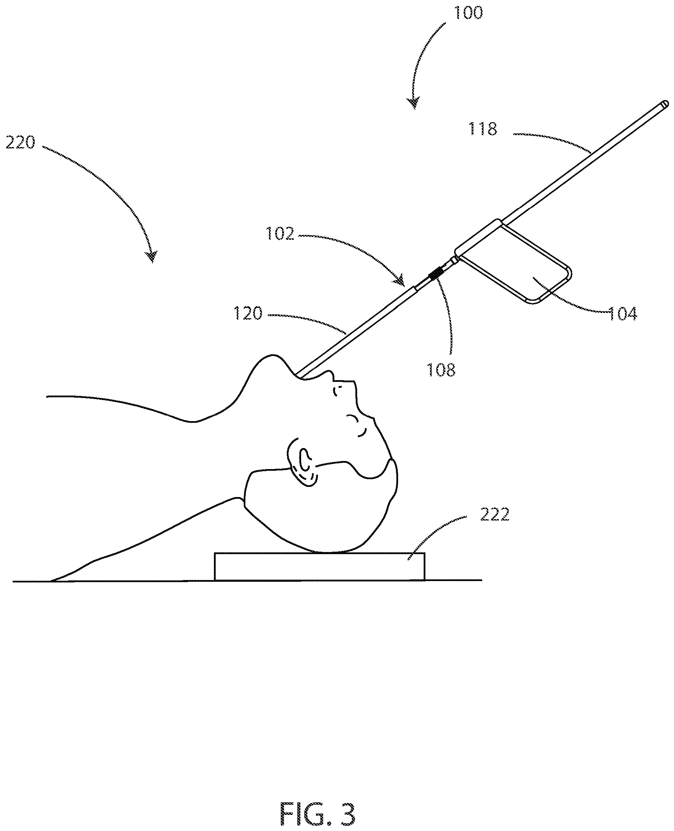

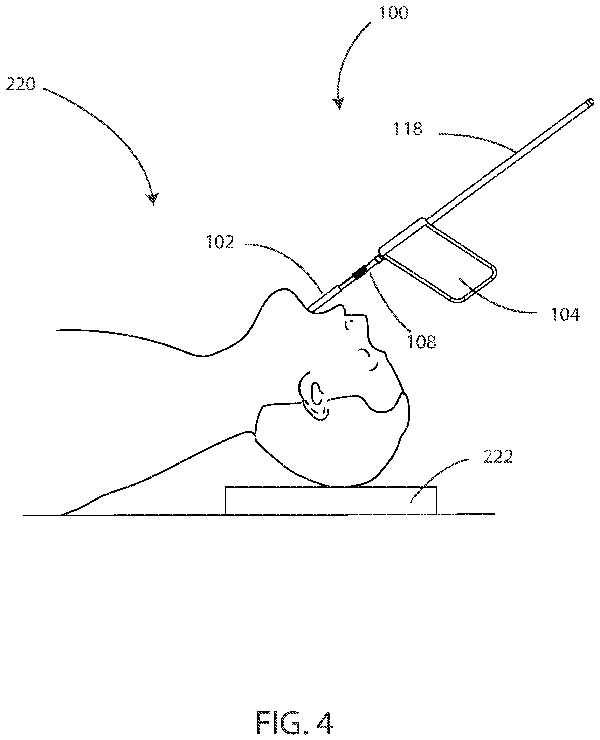

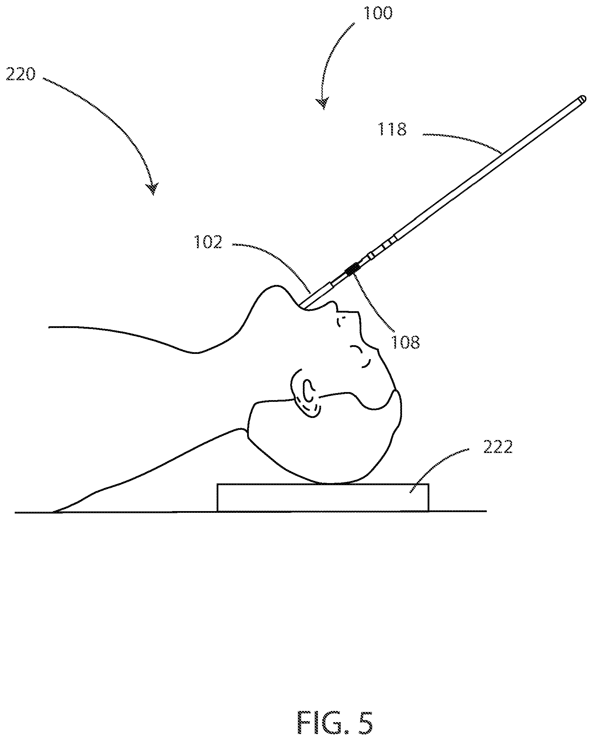

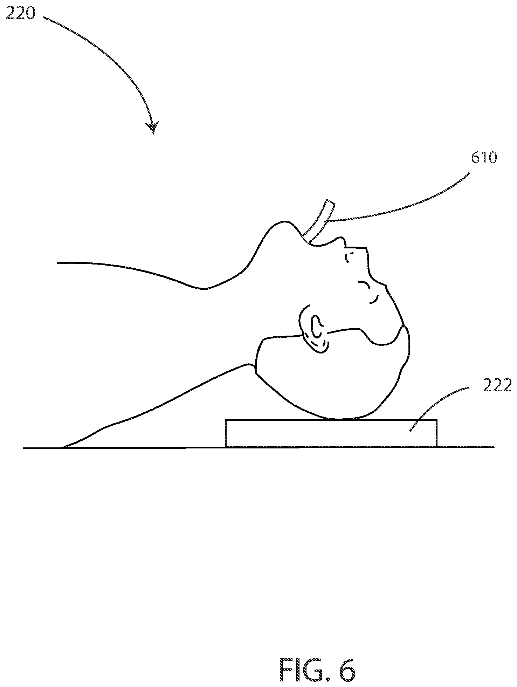

FIG. 3 is a drawing showing a patient an intubation tool 100 during initial insertion of the distal end of stylet 102 inserted through the mouth of the patient. FIG. 4 shows the stylet 102 extended further into the patient; while FIG. 5 shows the stylet 102 within a patient and the handle removed so that the stylet 102 remains, with proximal portion 118 extending out of the patient's mouth and ready for placement of the intubation tube (not shown). FIG. 6 is a drawing showing a patient with an endotracheal tube 610 emplaced.

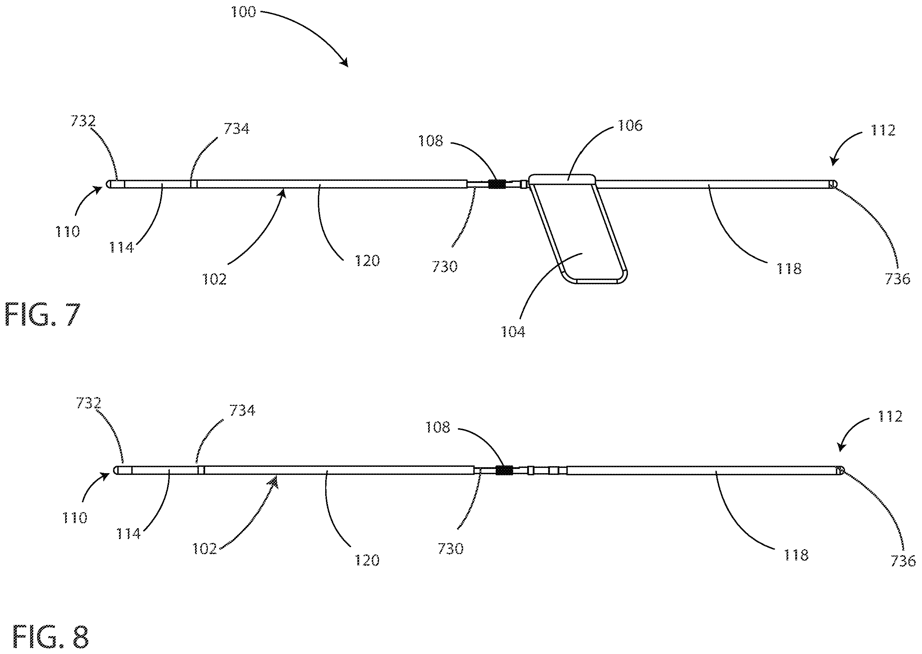

FIG. 7 is a drawing showing a side plan view of the endotracheal intubation tool 100 for assisting in placement of an endotracheal tube made in accordance with an example embodiment, showing the stylet of the tool with a removable handle 104 clipped in place. FIG. 8 is a drawing showing a side plan view of endotracheal intubation tool 100 the handle removed. The intubation tool 100 includes a stylet 102 having distal end 110 and proximal end 112 that is flexible and has an articulating tip 114 that allows for precise placement during intubation of a patient, thereby allowing placement with minimal disturbance or damage to the patient. The stylet 102 further includes a steering base 730 onto which a steering control 108 is mounted. The steering control 108 is joined to a cable (not shown) that travels down distal portion 120 of the stylet, through articulation collar 734 and to steering cable tip 732, where the ends of the steering cable are secured. By moving the steering control 108 forward and backward (using as little as one, two, or three fingers (for example)) the articulating tip is articulated (or bent) by forces delivered by the cable connecting the steering control 108 to steering cable tip 732. Stylet 102 further includes a tube end cap 736 on the proximal end of the stylet 102. It is this tube end cap 736 over which the distal end of an intubation tube passes when the intubation tube is passed over the stylet during intubation.

Removal of the handle 104 is accomplished in the depicted embodiment by opening clasp 106, as shown later herein. The stylet 102 includes a steering control 108 that can slide forward and backward so as to change the shape of articulating tip 114. Stylet 102 further includes a proximal portion 118 and a distal portion 120, both of which are typically flexible but not articulating. Proximal portion 118 of stylet 102 remains outside of a patient during the intubation procedure, while at least some of distal portion 120 is inserted into the patient.

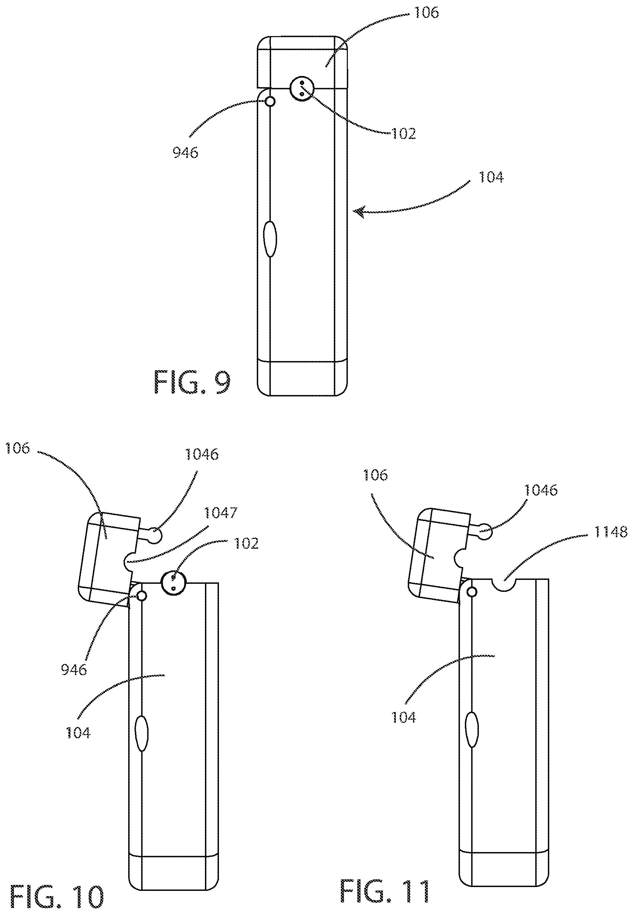

FIG. 9 is a front view of the distal side of a handle 104 of an intubation tool with the stylet 102 of the tool installed; while FIG. 10 is a front view of the distal side of the handle 104 of the intubation tool with the clasp 106 open, and FIG. 11 is a front view of the distal side of the handle 104 of the intubation tool with the tool removed and handle catch open. FIG. 9 shows a dowel 946 (typically made of metal, such as stainless steel) that the clasp 106 pivots around. In addition, the clasp 106 shows a locking member 1046 for snapping into the lower portion of the handle 104. The clasp 106 further includes a recess 1047, while the bottom of the handle 104 includes a recess 1148. Recess 1047 and 1148 combine to surround and hold the stylet 102 when the handle 104 is retained on the stylet 102.

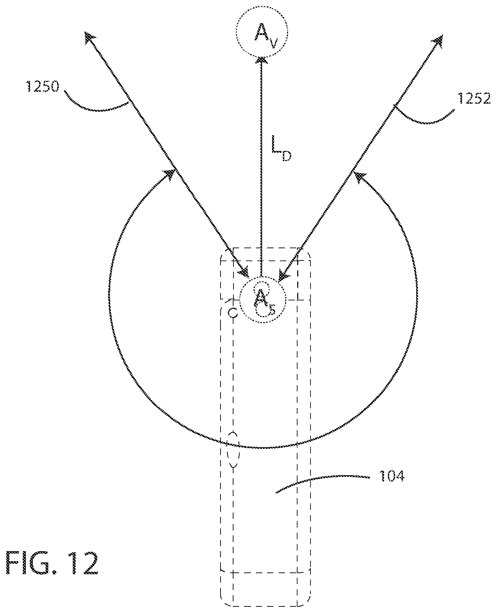

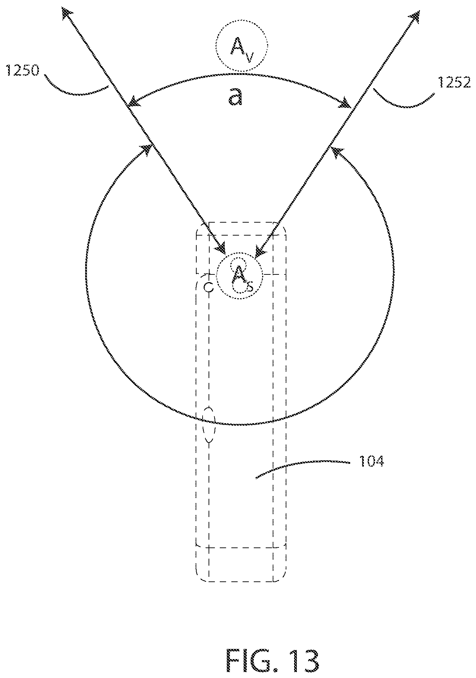

FIG. 12 is a diagram showing the field of view from intubation tool made in accordance with the present disclosure. FIG. 13 is a diagram showing the field of view from intubation tool made in accordance with the present disclosure. Both figures show how the intubation tool is designed to allow for improved visibility into a patient during medical procedures. Generally, a relatively unobstructed view is possible relative to alternative devices. In an embodiment, the handle and stylet provide a substantially clear sight line of at least 180 degrees along the dorsal line of the handle and stylet combination. In an embodiment, the handle and stylet provide a substantially clear sight line of at least 90 degrees along a dorsal line of the handle and stylet combination, within 1 cm of the central axis of the stylet. In an embodiment, the handle and stylet provide a substantially clear sight line of at least 90 degrees along a dorsal line of the handle and stylet combination, within 2 cm of the central axis of the stylet. In an embodiment, the handle and stylet provide a substantially clear sight line of at least 270 degrees along a dorsal line of the handle and stylet combination, within 1 cm of the central axis of the stylet.

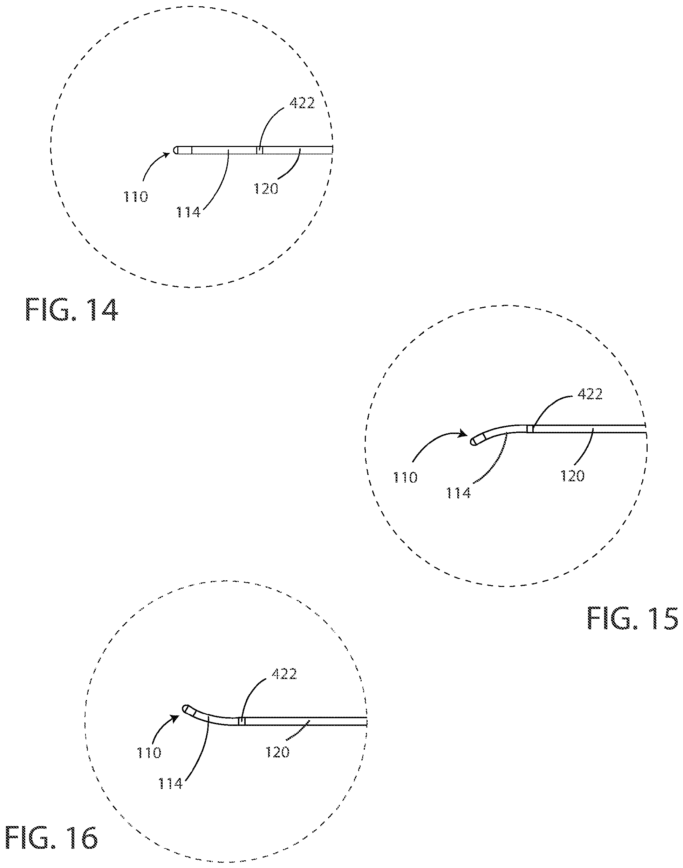

FIG. 14 is view of the distal end of an intubation tool stylet, showing the articulating tip 114 of the stylet 102 in a neutral position; FIG. 15 is view of the distal end of the stylet, showing articulating tip 114 in an downwardly flexed position; FIG. 16 is view of the distal end of stylet 102, showing the tip of the stylet in an upwardly flexed position. In this neutral position the distal end allows for insertion into a patient without trauma to the patient's tissue.

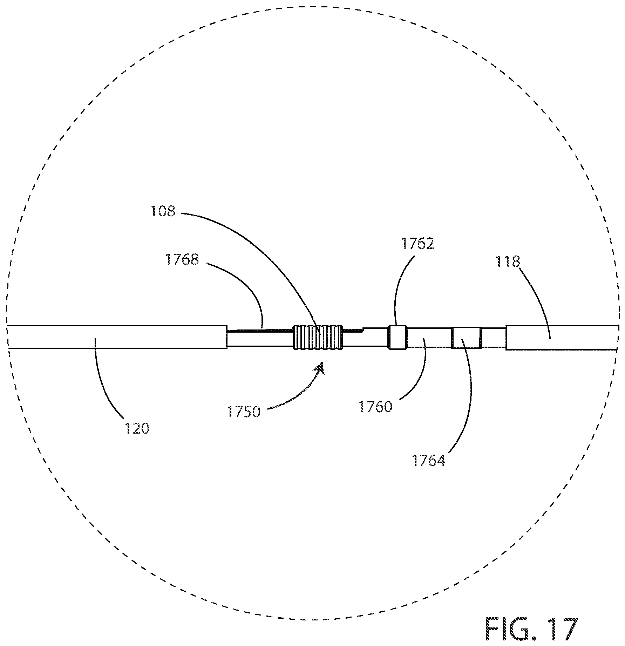

FIG. 17 is a closeup view of the steering base of an intubation tool stylet 102. The steering base 1750 includes steering control 108 that readily moves to a distal (left) location or proximal (right) location with the force of one or more fingers. The steering base 1750 includes a path 1768 on which the steering control 108 travels. This path 1768 is generally exposed even when the stylet 102 is mounted on a handle (not shown), allowing the operator to move the steering control 108 so as to articulate the tip of the stylet 102. Further, a proximal section 1760 of the steering base 1750 is shown, this proximal section 1760 including (in this embodiment) regions 1762 and 1764 that are wider areas of the steering base 1750 deigned to secure the stylet 102 in a handle and prevent rotation and sliding of the stylet.

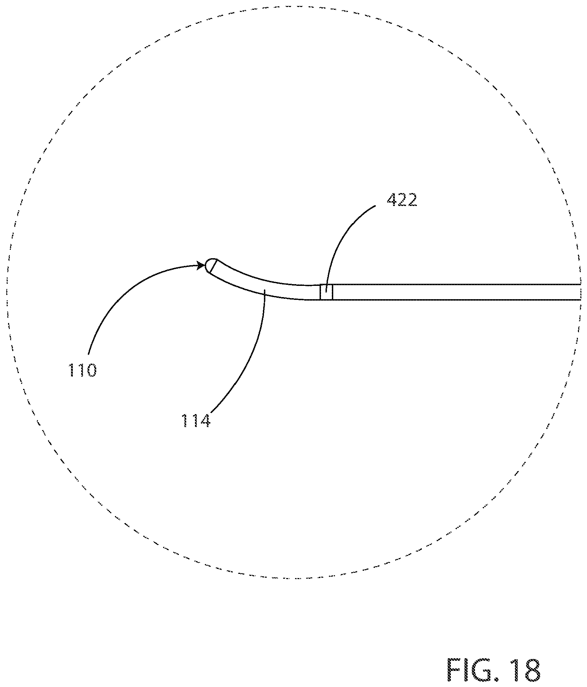

FIG. 18 is view of the distal end of an intubation tool stylet, with the tip of the stylet articulated.

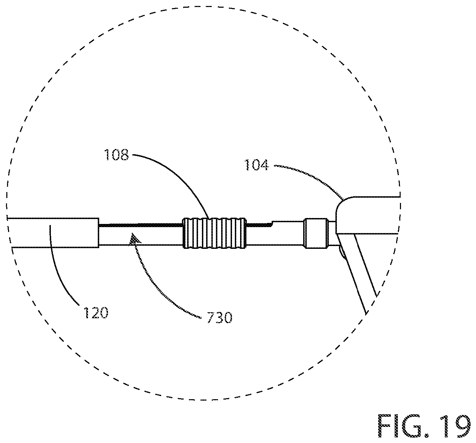

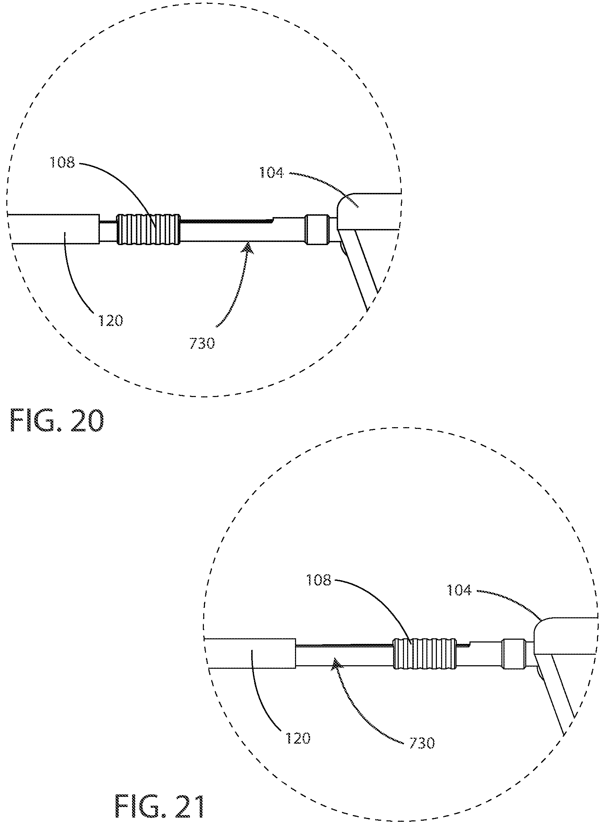

FIG. 19 is close up view of the medial portion of a stylet 102, showing the steering control 108 in a substantially neutral location; FIG. 20 is close up view of the medial portion of a stylet 102, showing the steering control 108 in a substantially distal location; FIG. 21 is close up view of the medial portion of a stylet 102, showing the steering control 108 in a substantially proximal location.

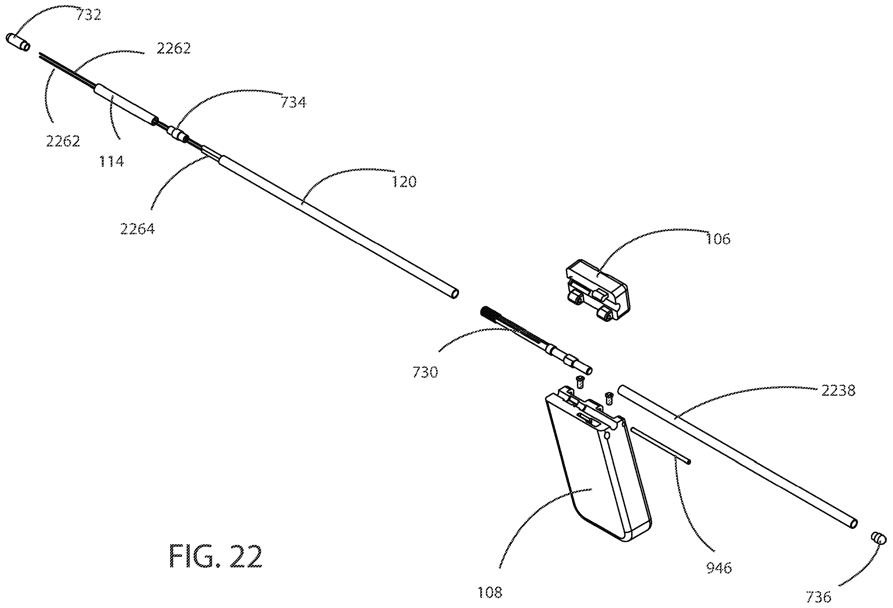

FIG. 22 is a drawing showing an exploded view of components of an intubation tool for assisting in placement of an endotracheal tube made in accordance with an example embodiment; further showing two ends of steering cable 2262 and also optional bowden cable 2264 through which the steering cable 2262 passes. The steering cable 2262 terminates in the steering cable tip 732, while also being connected to steering control 108.

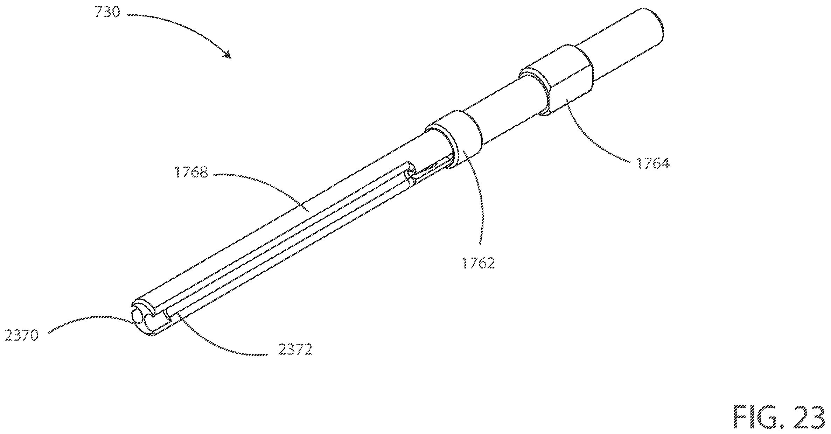

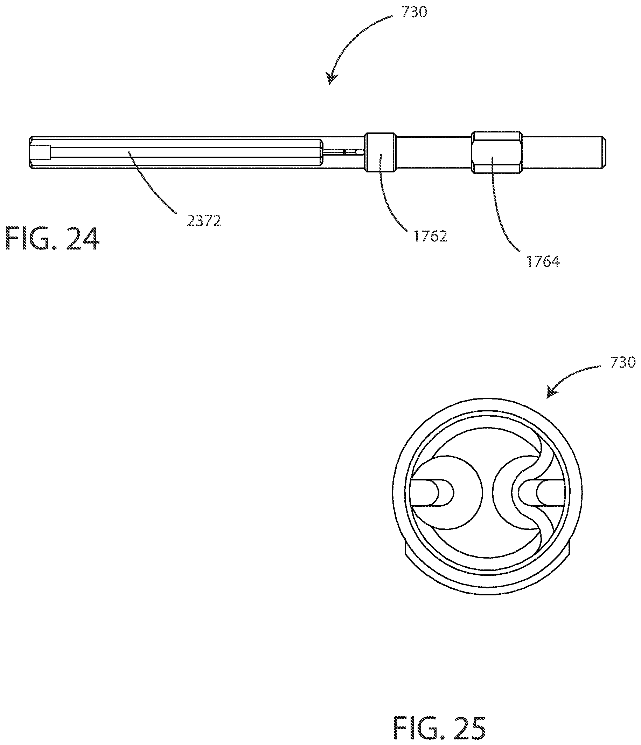

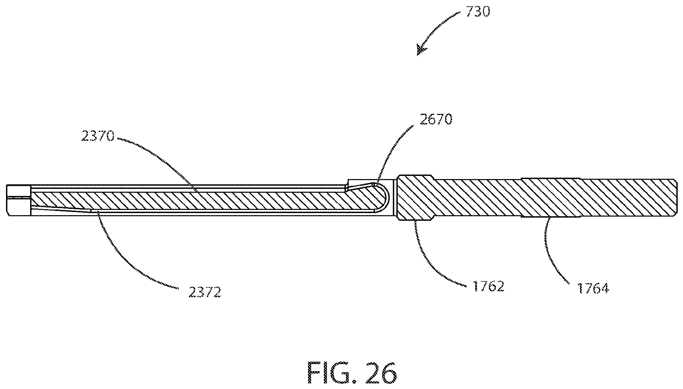

FIG. 23 is a perspective view of a steering base 730 made in accordance with an example embodiment; FIG. 24 is a side elevational view of the steering base 730; FIG. 25 a distal end view of a steering base 730; and FIG. 26 is a cross sectional view of the steering base 730. Channels 2370 and 2372 are shown in steering base 730, and these channels provide pathway 2670 for the steering cable 2262. Sleeve 2238 on the proximal end the stylet is further shown.

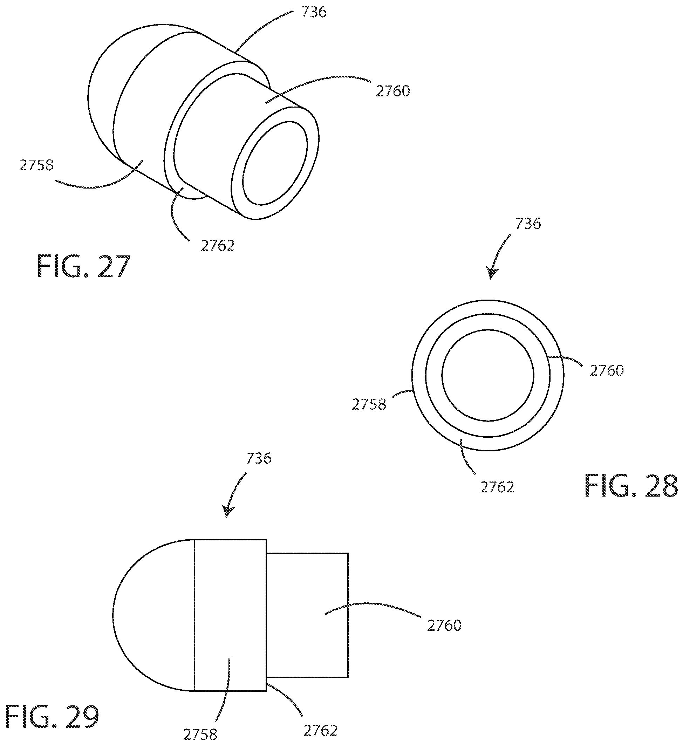

FIG. 27 is a perspective view of the proximal end cap 736 of an intubation tool stylet in accordance with an embodiment; FIG. 28 is a distal end view of the proximal end cap 736; and FIG. 29 is a side view of the proximal end cap. Exposed portion 2758 is shown, along with recessed portion 2760 for insertion into the proximal end of the stylet, along with lip 2762 to limit insertion depth of the proximal end cap.

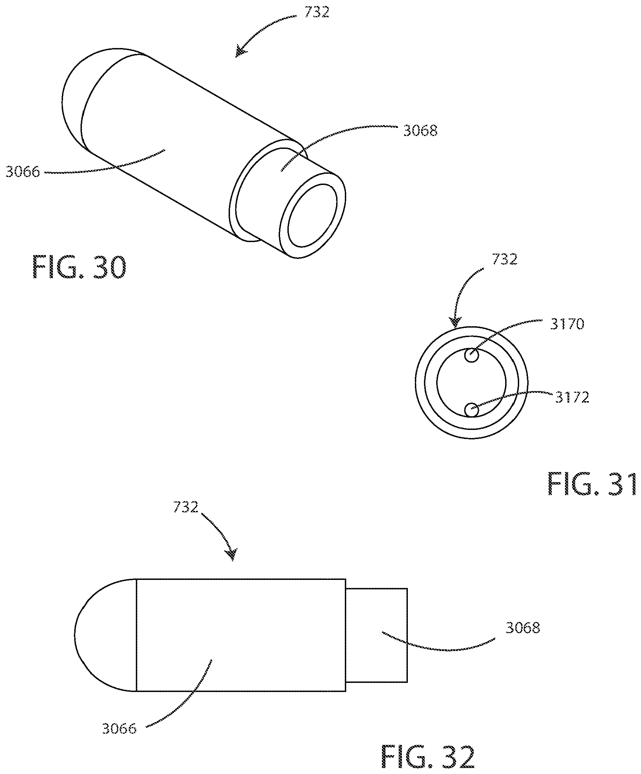

FIG. 30 is a perspective view of steering cable tip 732 of an intubation tool stylet in accordance with an embodiment; FIG. 31 is a proximal end view of steering cable tip; and FIG. 32 is a side view. Exposed portion 3066 is shown, along with recessed portion 3068 as are cable retaining holes 3170 and 3172 are shown.

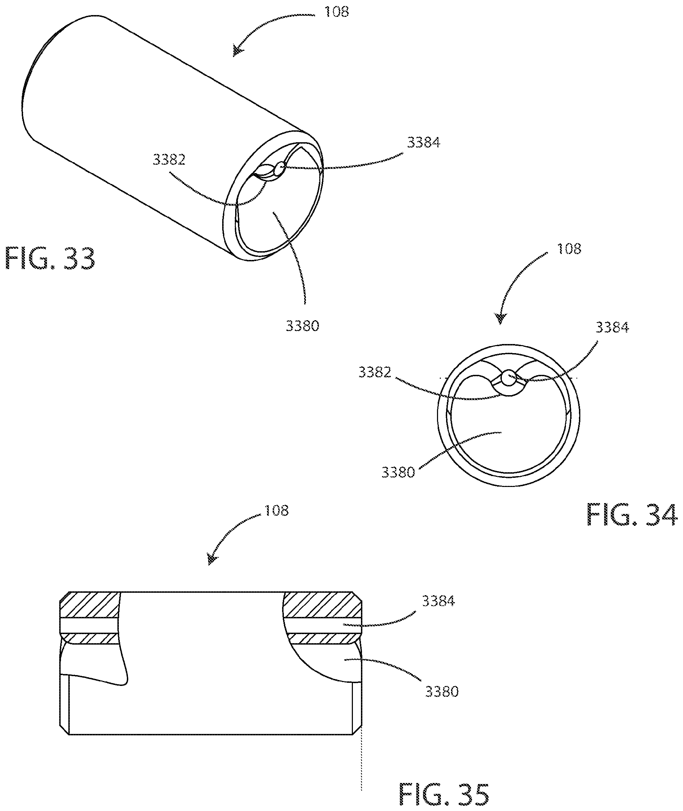

FIG. 33 is a perspective view of steering control 108 showing an internal opening 3380 that fits onto steering base 730. The steering control 108 further includes a protrusion 3382 that fits into a recess in the steering base 730, thereby preventing rotation of the steering control 108. In addition, an opening 3384 is formed in the protrusion 3382, and this opening secures the cable connecting to the articulating tip of the stylet; FIG. 34 is an end view of the steering control 108; and FIG. 35 is a side view of the steering control 108.

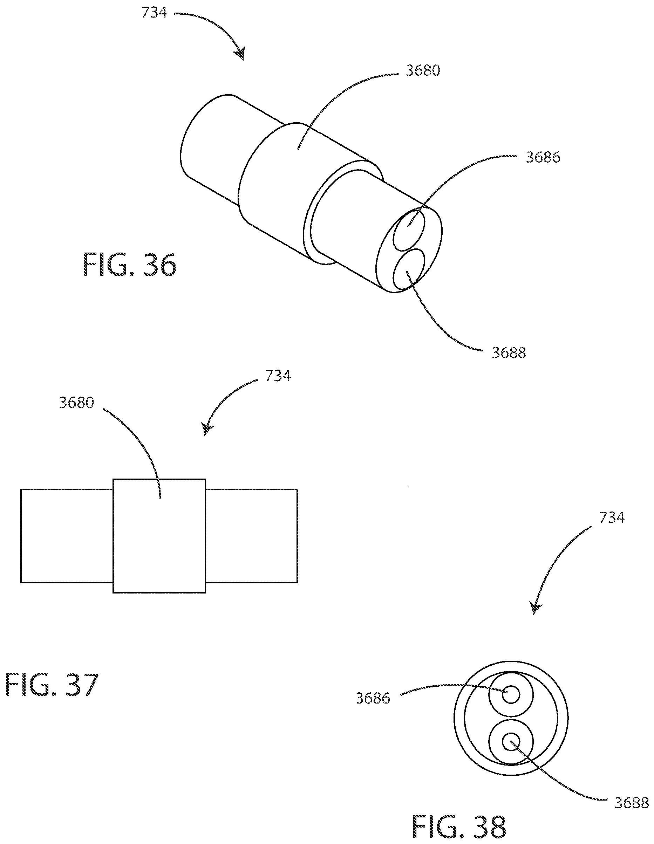

FIG. 36 is a perspective view of an articulation collar 3680 of a stylet in accordance with an embodiment; and FIG. 37 is a side view of the articulation collar 734; FIG. 38 is an end view of the articulation collar3680, having pathways 3686 and 3688.

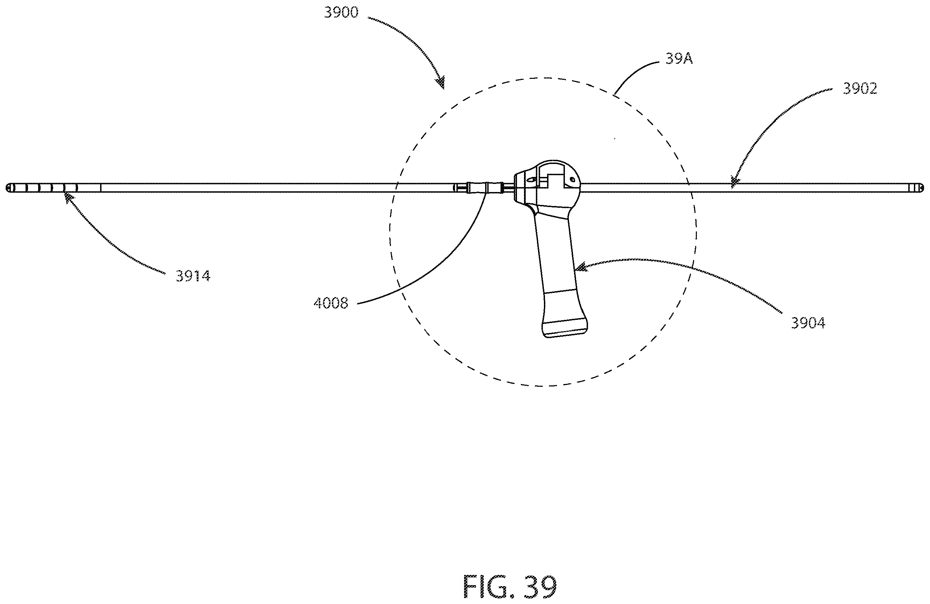

FIG. 39 is a drawing showing a side plan view of endotracheal intubation tool 3900 for assisting in placement of an endotracheal tube made in accordance with an example embodiment. The intubation tool 3900 includes a stylet 3902 and a handle 3904 having an articulating tip 3914 and a steering control 4008. The steering control 4008 slides forward and backward (distally and proximally) along the stylet 3902, and this movement of the steering control 4008 moves the tip of the stylet upward and downward (dorsally and ventrally).

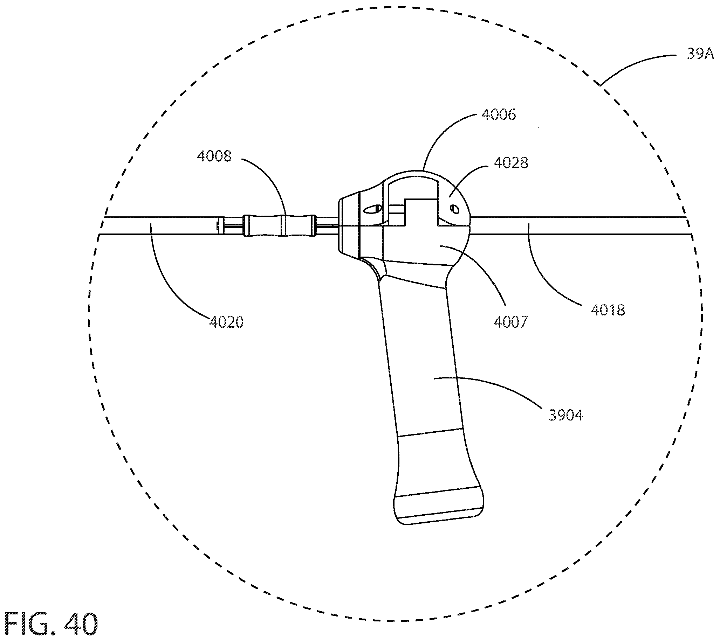

FIG. 40 is closeup of the endotracheal intubation tool of FIG. 39, enlarging the portion from dotted circle 39A. FIG. 40 shows the distal end 4020 of the stylet and the proximal end 4018 of the stylet, as well as clasp 4006 on handle 3904, along with base 4007 of handle 3904. The stylet is secured between the clasp 4006 and the base 4007 during placement of the stylet into a patient. After placement of the stylet into the patient the clasp is opened or removed, freeing the stylet from the handle 3904 so that an intubation tube may be inserted over the stylet. During use rounded surface 4028 of the handle 3904 is typically placed within the palm of the medical professional performing the intubation procedure, and the medical professional fingers wrap around the top of the handle 3904 to grasp the steering control 4008. Typically 2 or 3 fingers make contact with the steering control 4008 so as to pinch the steering control 4008 between the fingers. Minimal force is thereafter needed to move the steering control forward and backward to articulate the tip of the stylet (not shown). Thus, merely drawing the fingers forward and backward as they contact the stylet, typically in a pinching arrangement with two or more fingers arranged around the steering control 4008, results in articulation of the tip of the stylet.

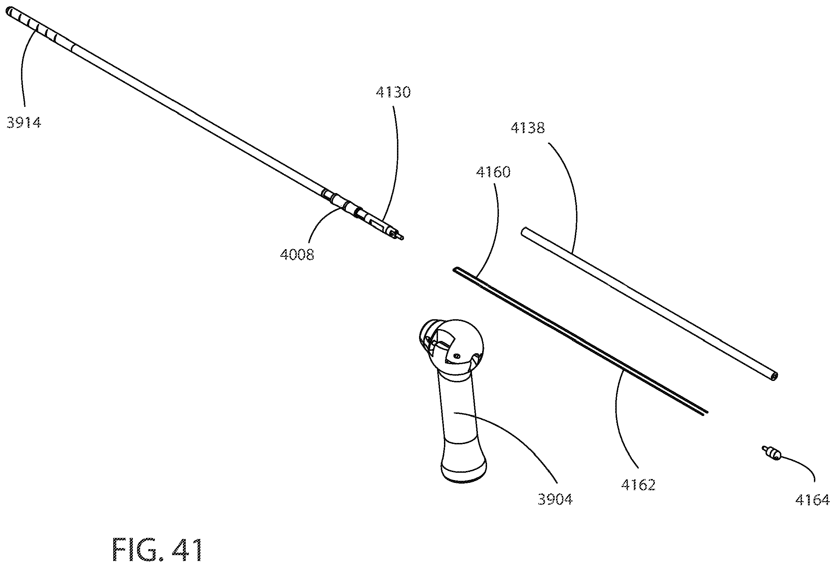

FIG. 41 is a drawing showing an exploded view of components of an endotracheal intubation tool for assisting in placement of an endotracheal tube made in accordance with an example embodiment. The components include handle 3904, articulating tip 3914, steering control 4008 positioned on a steering base 4130. In the depicted embodiment, two lengths of cable 4160 and 4162 which extend through the proximal end of the stylet through proximal sleeve 4138. Tip 4164 is at the most proximal end of the stylet.

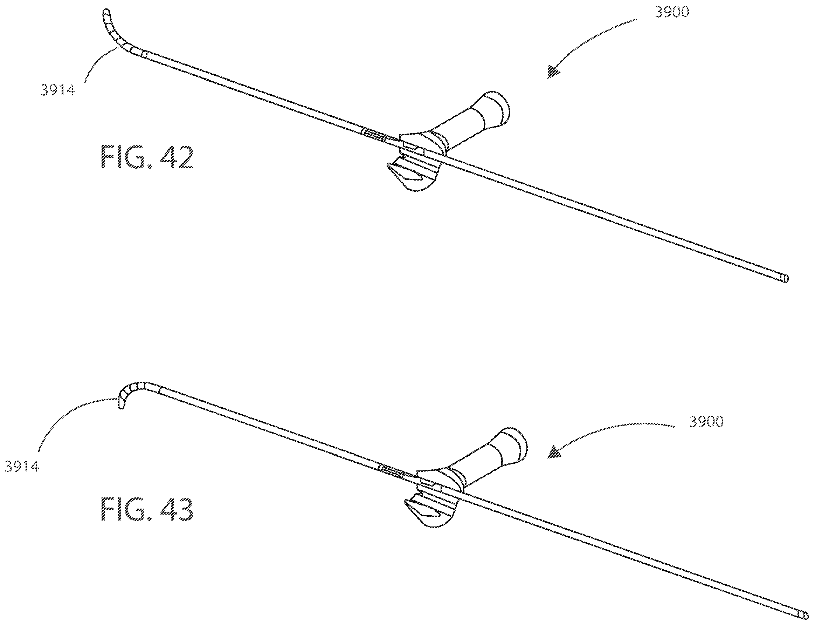

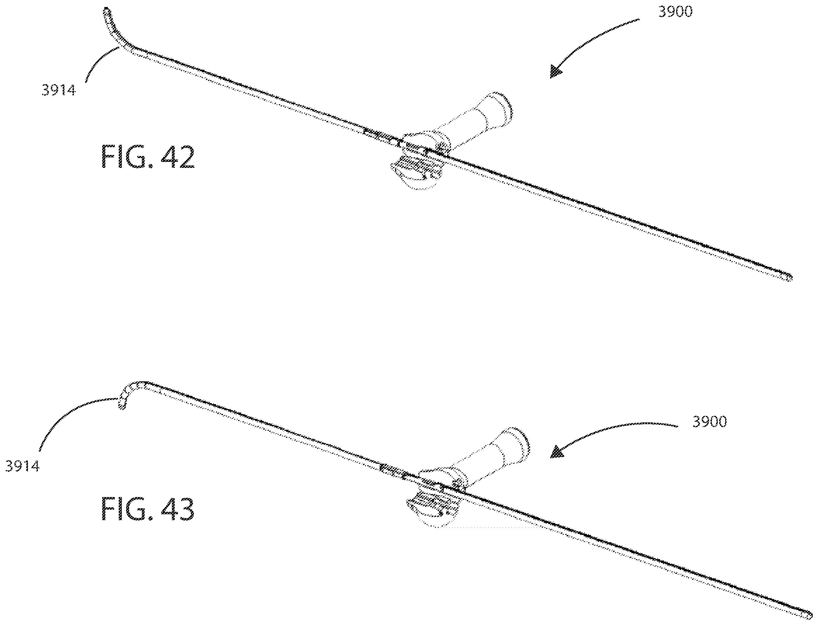

FIG. 42 is a drawing showing a side perspective view of endotracheal intubation tool 3900 for assisting in placement of an endotracheal tube made in accordance with an example embodiment, showing the articulating tip in an upward (or dorsal) articulation. In the embodiment shown the articulating tip 3914 is shown in a position where the tip is articulated in a dorsal (or upward) orientation. In the depicted embodiment the upward orientation occurs as the steering control 4008 (see, e.g., FIGS. 40 and 41) is drawn in a proximal direction. FIG. 43 is a drawing showing a side perspective view of the endotracheal intubation tool of FIG. 42, with the articulating tip 3914 in a ventral (or downward) orientation. In the depicted embodiment the downward orientation occurs as the steering control 4008 is moved in distal direction.

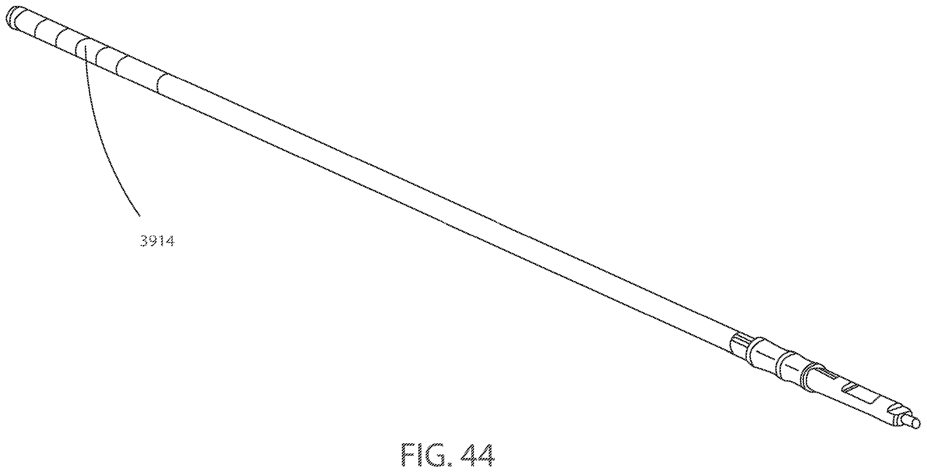

FIG. 44 is drawing showing the articulating tip 3914 of an endotracheal intubation tool in a neutral position. In general, the articulating tip 3914 is in a neutral (or straight) position when no forced is being applied to the tip, such as from the steering control 4008.

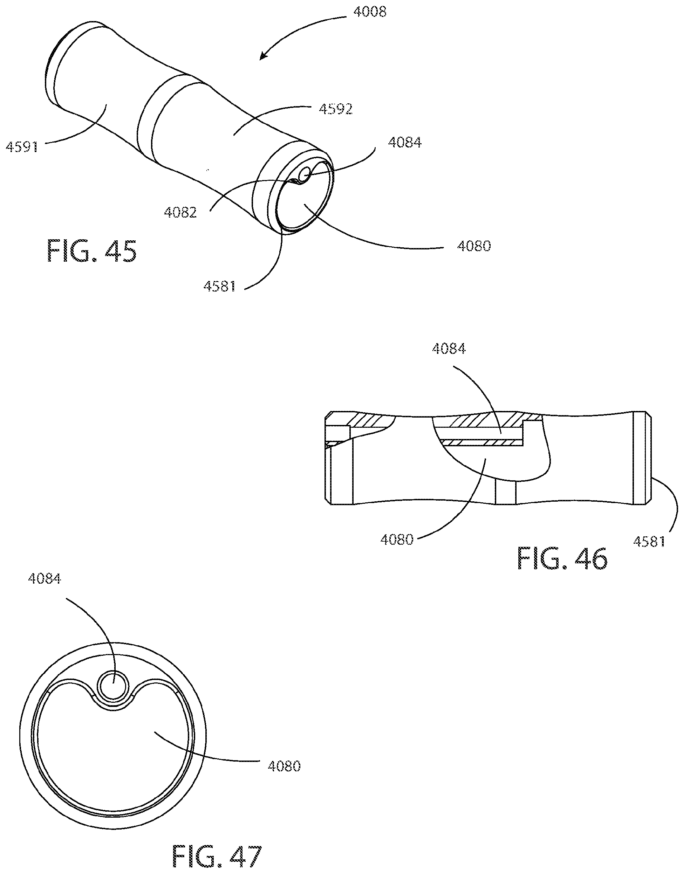

FIG. 45 is a perspective view of steering control 4008 of an intubation tool stylet in accordance with an embodiment, the steering control 4008. Steering control 4008 has internal opening 4080 that fits onto steering base. The steering control 4008 further includes a protrusion 4082 that fits into a recess in the steering base, thereby preventing rotation of the steering control 108. Steering control 4008 also includes an opening 4084 for securing the cable connected to the articulating tip of the stylet. The steering control 4008 has to recessed portions 4591 and 4592 for gripping by fingers (although other configurations are possible). Lip 4581 of the steering control 4008 is also shown. FIG. 46 is a side view of steering control 4008 of an intubation tool stylet in accordance with an embodiment, with a partial cross section. FIG. 47 is an end view of steering control 4008 of an intubation tool stylet in accordance with an embodiment.

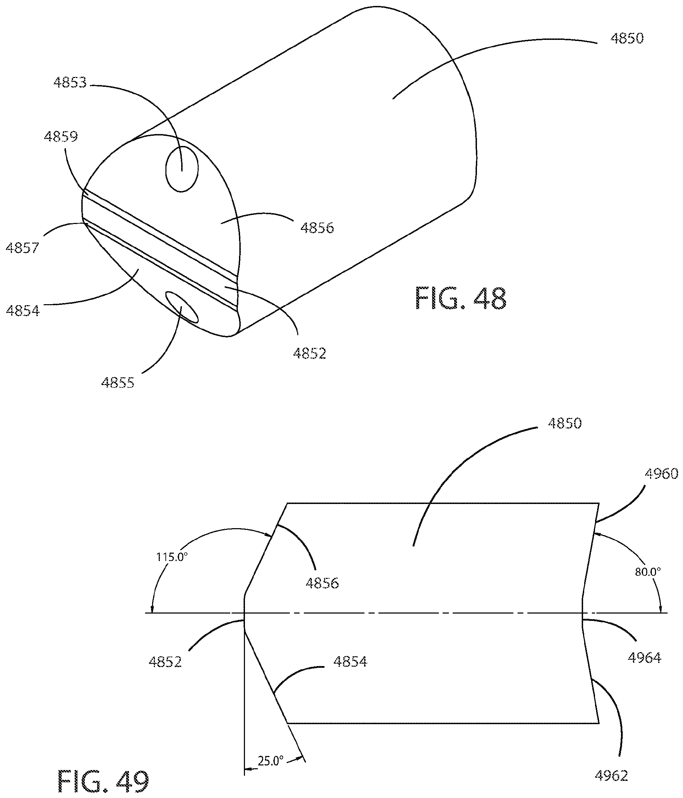

FIG. 48 is a perspective view of a vertebrate 4850 from the articulating end of an articulating tip, showing the vertebrate 4850 independent of other components. The vertebrate 4850 has a first distal angled face 4854 and a second distal angled face 4856 separated, in this embodiment, by a distal flat portion 4852, along with two optional transition regions 4857 and 4859. The vertebrate also includes internal pathways 4853 and 4855 through which the cables are passed to secure multiple vertebrate together and to apply tension to articulate the vertebrate relative to one another.

FIG. 49 is a side view of a vertebrate 4850 from the articulating end of an articulating tip, showing the vertebrate independent of other components. The first angled face 4854 and second angled face 4856 are shown, along with flat portion 4852. These distal angled faces 4854, 4856 and distal flat portion 4852 are on the distal end of the vertebrate (that portion closest to the patient). FIG. 49 further shows the proximal end of the vertebrate, including a first proximal angled face 4960 and a second proximal angled face 4962, and a proximal flat portion 4964.

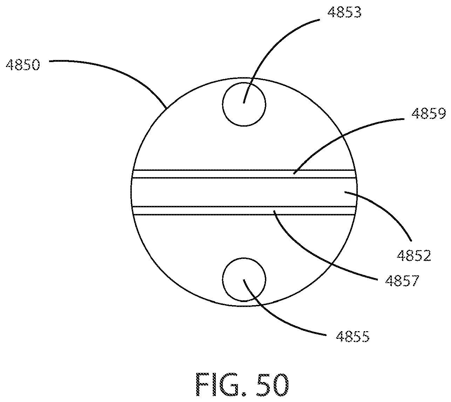

FIG. 50 is a distal end view of a vertebrate 4850 from the articulating end of an articulating tip, showing the vertebrate 4850 independent of other components. The vertebrate 4850 has a first distal angled face 4854 and a second distal angled face 4856 separated, in this embodiment, by a distal flat portion 4852, along with two optional transition regions 4857 and 4859. The vertebrate also includes internal pathways 4853 and 4855 through which the cables are passed to secure multiple vertebrate together and to apply tension to articulate the vertebrate relative to one another.

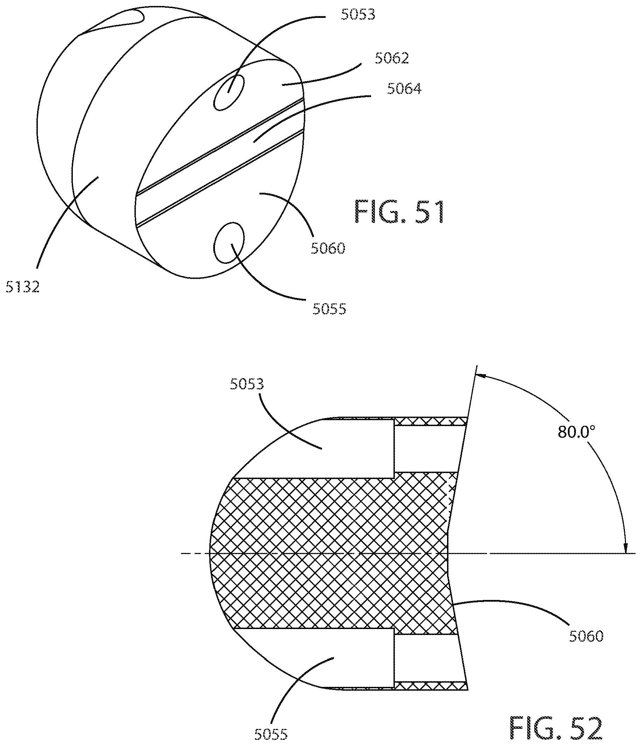

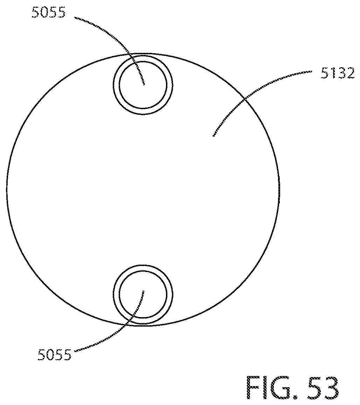

FIG. 51 is a perspective view of the tip member 5132 from an end of an articulating tip, showing the tip member 5060 independent of other components. The tip member 5132 has a first proximal angled face 5060 and second proximal angled face 5062, plus a proximal flat portion 5064. Passageways 5053 and 5055 allow for placement of ends of the control cable (not shown) which are typically secured by adhesive or other means in the end of the passageways. FIG. 52 is a side cross-sectional view of the tip member 5060 from an articulating end of an articulating tip, showing the tip member independent of other components, and FIG. 53 is an end view of the tip member 5060.

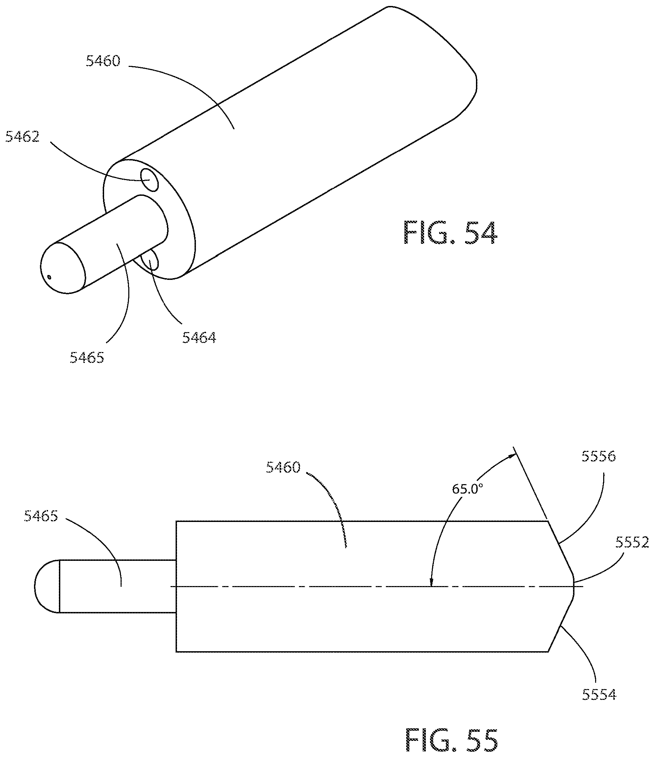

FIG. 54 is a perspective view of a base member 5460 from the proximal end of an articulating tip, showing the base member independent of other components; and FIG. 55 is a side view of the base member 5460. The base member 5460 includes passageways 5462 and 5464, and also include a stem 5465 that can fit within a hole in the shaft of the stylus to secure it into place. The distal end of the base member 5460 includes distal angled face 5554 and second distal angled face 5556, separated by a distal flat portion 5552

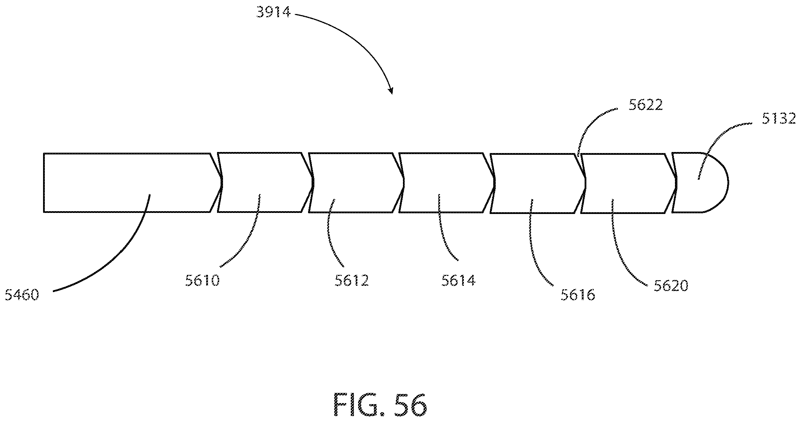

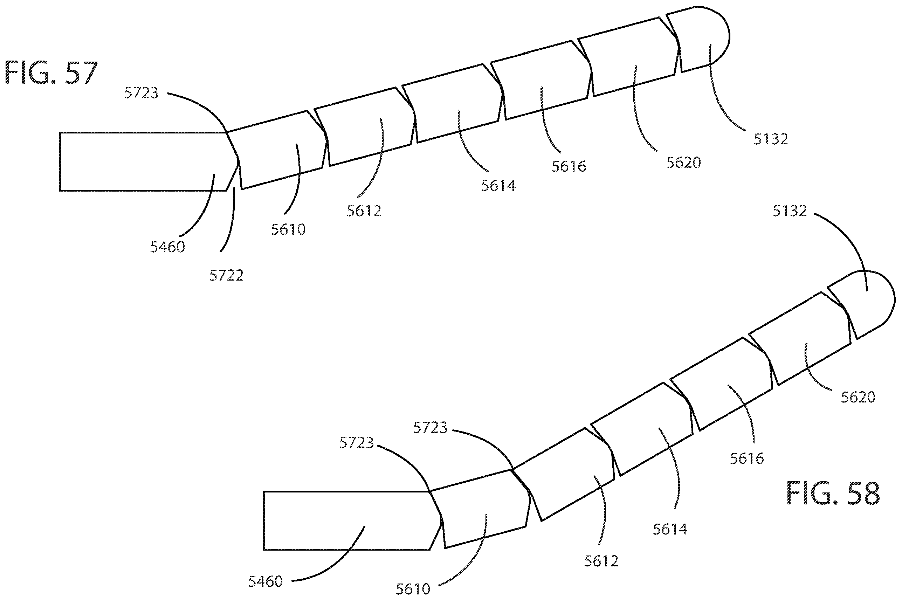

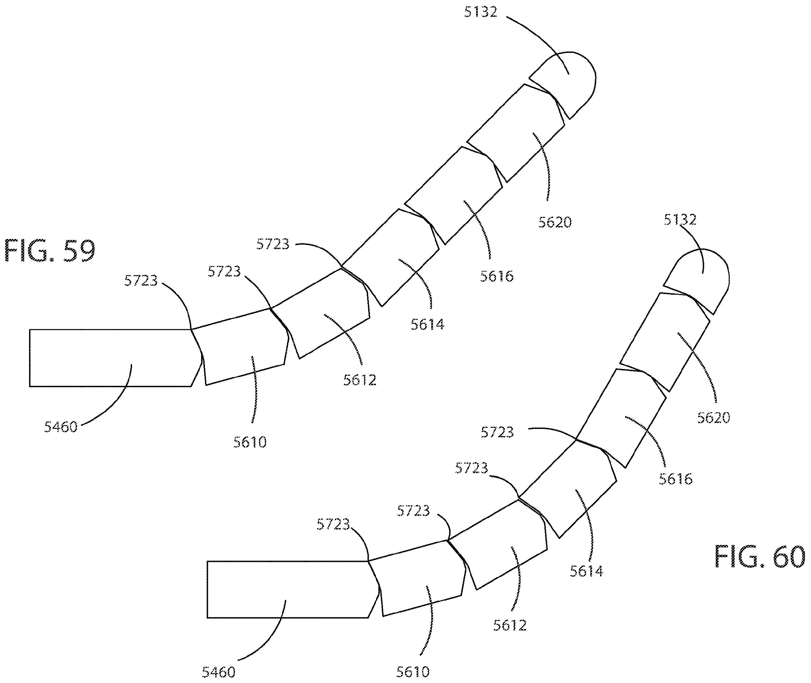

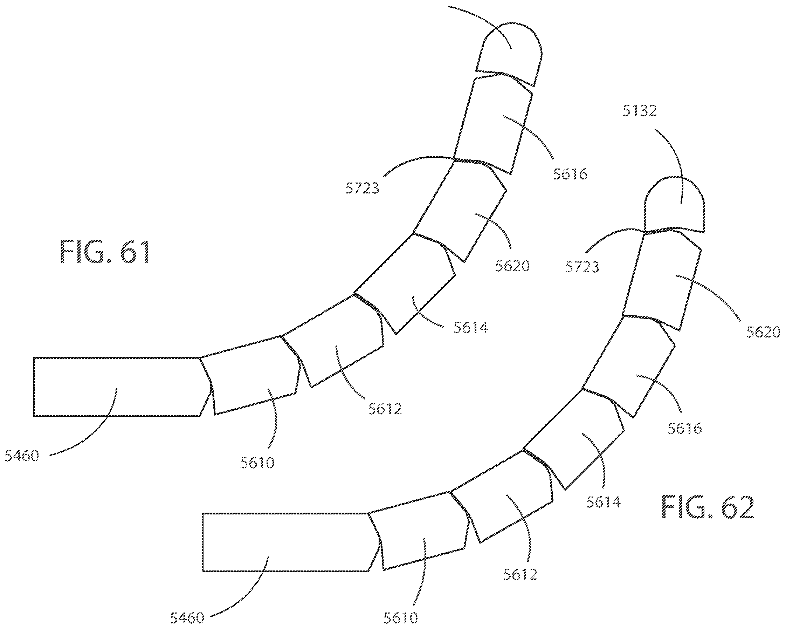

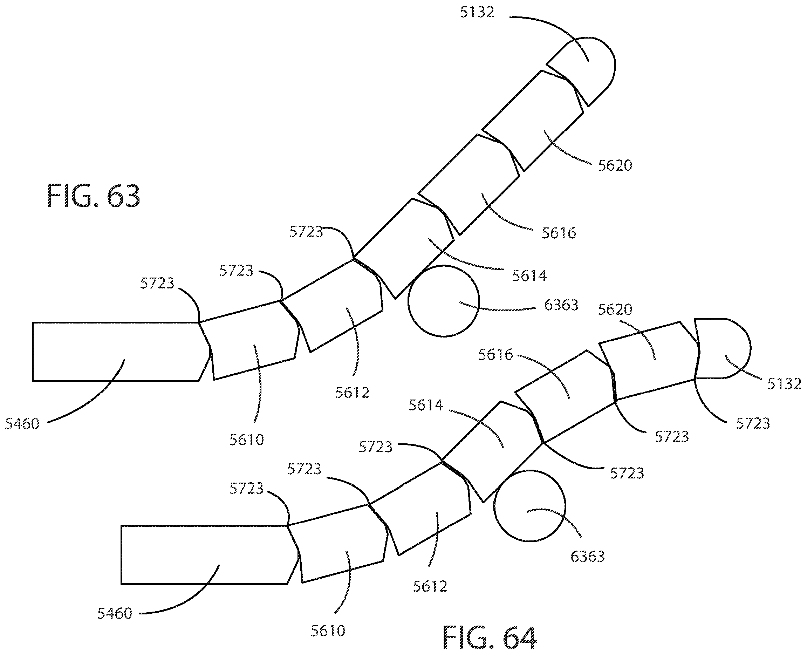

FIG. 56 is a side elevational view of components of an articulating tip of an endotracheal intubation tool in a neutral position, showing a base member 5460, multiple vertebrate 5610, 5612, 5614, 5616, and 5620, along with a tip member 5132. A gap 5622 is shown between the vertebrate 5616 and 5620. FIGS. 57 to 62 show the articulating tip in increasing levels of articulation. As the vertebrate start to articulate the gaps 5622 close into contact regions 5723. On the opposite side an opening 5722 becomes larger. The proximal vertebrate articulate first, followed sequentially down the articulating tip to the most distal vertebrate and the tip member 5132. FIGS. 63 and 64 show the articulating around a stylized obstruction 6363, such as a portion of a patient's anatomy.

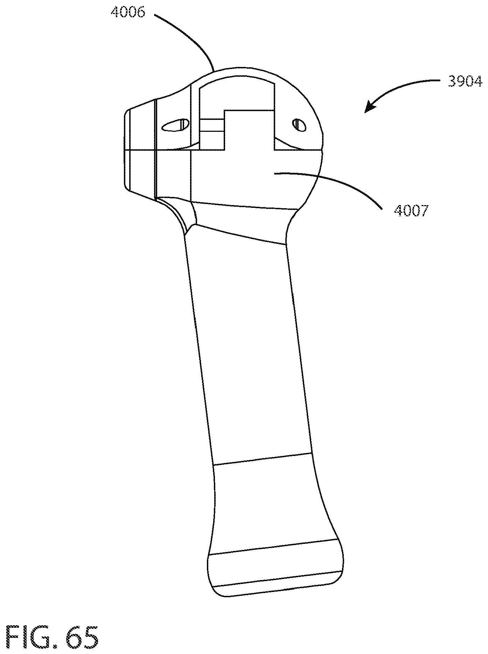

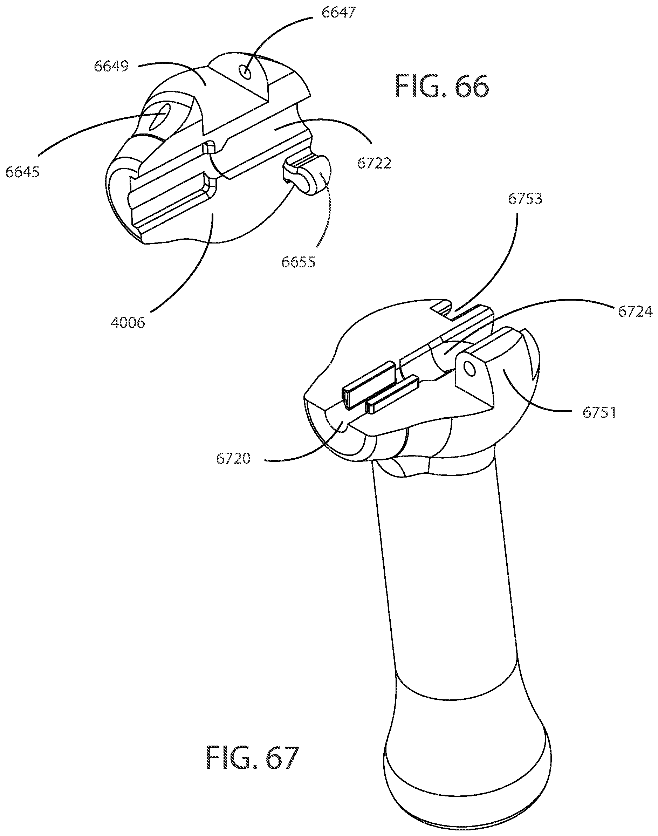

FIG. 65 is a side view of a handle 3904 of an endotracheal intubation tool with the stylet of the tool removed, showing a base 4007 and clasp 4006. FIG. 66 is a perspective view of a clasp 4006 of a handle, showing a protrusion 6655 for fitting into a recess 6753 of a base of a handle shown in FIG. 67 to snap the clasp and base together. A recess 6722 in the clasp combines with a recess 6720 in the base to form a passageway through the handle for the stylet. A hinge for the clasp and base is formed from an elevated portion 6751 of the base that fits into a slot 6649 in the clasp, and a pin can be inserted through holes 6645 and 6647 through the elevated portion 6751 of the base. Also shown is a recess 6724 that allows for the stylet to be bent downward and out of the way of the user while gripping the handle 3904

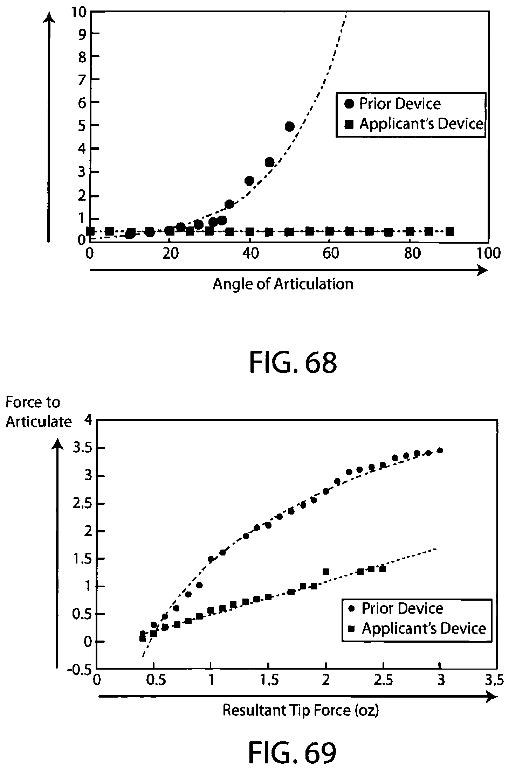

FIG. 68 is a graph showing force relative to articulation position of an articulating tip of an endotracheal intubation tool.

FIG. 69 is a graph showing force relative to articulation position of an articulating tip of an endotracheal intubation tool.

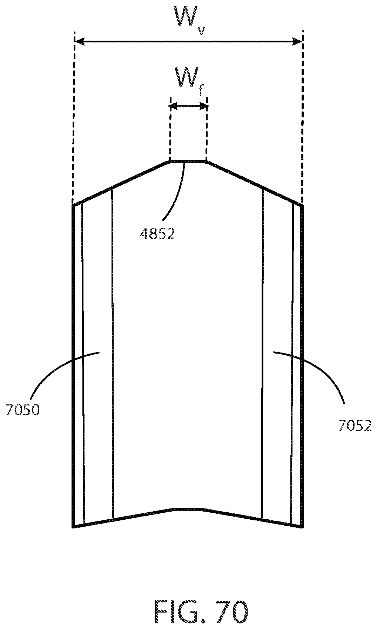

FIG. 70 is a side cross-sectional of a vertebrate from an articulating end of an articulating tip, showing passageways 7050 and 7052. The vertebrate has a total width of Wᵥ, and the distal flat portion 4852 has a width of W_{f}.

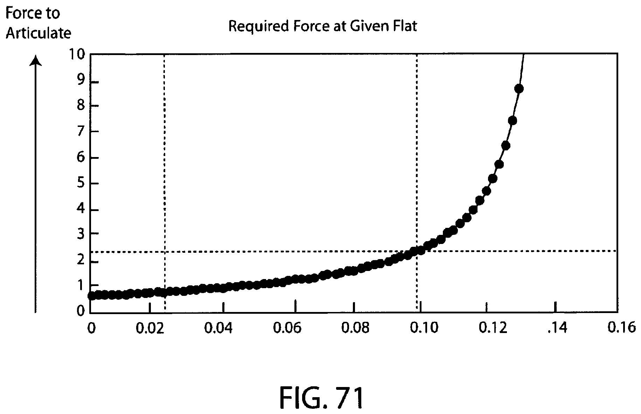

FIG. 71 is a graph showing required to articulate a vertebrate relative to flat portion of the vertebrate.

In an embodiment, a intubation tool for assisting in placement of an endotracheal tube, the intubation tool is included having a) a stylet having a proximal end, a distal end, and a medial location, the stylet: i) configured for receiving an endotracheal tube, and ii) having an articulating distal tip, the articulating distal tip being configured to articulate between more than one shape, and b) a removable handle secured to a medial location on of the stylet.

In an embodiment, wherein articulation of the distal tip of the stylet can be accomplished with a single hand that holds the removable handle and articulates the distal end.

In an embodiment, wherein articulation of the distal tip of the stylet can be accomplished with a single finger manipulating a steering control mounted on the stylet.

In an embodiment, wherein articulation of the distal tip of the stylet can be accomplished with a two fingers manipulating a steering control mounted on the stylet, the steering control sliding along the stylet.

In an embodiment, wherein articulation of the distal end of the stylet can be accomplished with three fingers from a single hand that articulates the distal end.

In an embodiment, the articulating distal tip can be articulated with a force of less than 9 Newton.

In an embodiment, the articulating distal tip can be articulated with a force of 4 to 9 Newton.

In an embodiment, the articulating distal tip is deformable upon contact with an obstruction.

In an embodiment, the articulating distal tip transmits force back to steering control upon contact with an obstruction.

In an embodiment, wherein translation movement of a steering control results in bending articulation of the articulating distal tip.

In an embodiment, wherein translation movement of a steering control by a distance D results in bending articulation of the distal tip by a distance of at least 150 percent of D.

In an embodiment, wherein translation movement of the steering control in a first direction results in bending articulation of the articulating distal tip in a first direction, and translational movement of the steering control in an opposite direction results in bending articulation of the articulating distal tip in a direction opposite the first direction.

In an embodiment, the stylet includes tubing containing at least one lumen.

In an embodiment, the stylet includes bilumen tubing.

In an embodiment, wherein upon removal of the handle from the medial portion of the stylet, the medial portion of the stylet has a diameter no greater than 120 percent of the remainder of the stylet.

In an embodiment, the widest portion of the stylet after removal of the handle is less than the diameter of the interior of the lumen of an endotracheal tube to be installed on the stylet.

In an embodiment, wherein handle and stylet provide a clear sight line of angle α of 20 degrees along a dorsal elevation LD of 1 cm.

In an embodiment, wherein handle and stylet provide a clear sight line of angle α of 20 degrees along a dorsal elevation LD of 2 cm.

In an embodiment, wherein handle and stylet provide a clear sight line of angle α of 20 degrees along a dorsal elevation LD of 3 cm.

In an embodiment, the handle and stylet provide a clear sight line of angle α of 90 degrees along a dorsal elevation LD of 1 cm.

In an embodiment, the handle and stylet provide a clear sight line of angle α of 180 degrees along a dorsal elevation LD of 3 cm.

In an embodiment, the handle and stylet provide a clear sight line of angle α of 270 degrees along a dorsal elevation LD of 3 cm.

In an embodiment, the stylet has a length at least 1.5 times the length of an endotracheal tube to be installed on the stylet.

In an embodiment, the stylet has a length at least 2.0 times the length of an endotracheal tube to be installed on the stylet.

In an embodiment, wherein handle and stylet provide a substantially clear sight-line along the length of the stylet when the stylet is in a non-articulated mode.

In an embodiment, wherein handle and stylet provide a substantially clear sight line of at least 180 degrees along the dorsal line of the handle and stylet combination.

In an embodiment, wherein handle and stylet provide a substantially clear sight line of at least 90 degrees along a dorsal line of the handle and stylet combination, within 1 cm of the central axis of the stylet.

In an embodiment, wherein handle and stylet provide a substantially clear sight line of at least 90 degrees along a dorsal line of the handle and stylet combination, within 2 cm of the central axis of the stylet.

In an embodiment, wherein handle and stylet provide a substantially clear sight line of at least 270 degrees along a dorsal line of the handle and stylet combination, within 1 cm of the central axis of the stylet.

In an embodiment, the handle is removable from the medial location of the stylet to allow placement of an endotracheal tube in place.

In an embodiment, the stylet articulates in a plane.

In an embodiment, the plane in which the stylet articulates can be selected by rotation the handle.

In an embodiment, the stylet includes an internal mechanism for articulating the distal end.

In an embodiment, the stylet includes an internal cable connected to the distal end, and wherein pulling on the cable from the medial location results in articulating the distal end of the stylet.

In an embodiment, further can include a steering element.

In an embodiment, the steering element includes a tube surrounding a portion of the medial location of the stylet.

In an embodiment, a method for placement of an endotracheal tube, the method is included, the method a) providing an intubation tool for assisting in placement of an endotracheal tube, the intubation tool is included, the method i) a stylet having a proximal end, distal end, and intermediate medial portion, the stylet configured for receiving an endotracheal tube, and having an articulating distal tip, the articulating distal tip being configured to articulate between more than one shape using one to three fingers, and ii) a handle secured to a medial position on the stylet, b) inserting the distal end of the stylet of the intubation tool into the trachea of a person, including articulating the distal end of the stylet during insertion to aid in passage through the larynx, c) removing the handle from the medial portion of the stylet, d) placing an endotracheal tube over the medial location of the stylet and down toward the distal end of the stylet and into the trachea of the person, and e) removing the stylet from the patient's trachea while keeping the endotracheal tube in place.

In an embodiment, the stylet articulates in a plane.

In an embodiment, the stylet includes an internal mechanism for articulating the distal tip.

In an embodiment, the stylet further includes a flexible intermediate portion.

In an embodiment, the stylet includes an internal cable connected to the distal tip, and wherein pulling on the cable from the medial location results in articulating the distal tip of the stylet.

In an embodiment, further can include an actuator.

In an embodiment, the actuator includes a tube surrounding a portion of the medial location of the stylet.

In an embodiment, wherein handle and stylet provide a substantially clear sight-line along the length of the stylet when the stylet is in a non-articulated mode.

In an embodiment, wherein handle and stylet provide a substantially clear sight line of at least 180 degrees along the dorsal line of the handle and stylet combination.

In an embodiment, wherein handle and stylet provide a substantially clear sight line of at least 90 degrees along a dorsal line of the handle and stylet combination, within 1 cm of the central axis of the stylet.

In an embodiment, wherein handle and stylet provide a substantially clear sight line of at least 90 degrees along a dorsal line of the handle and stylet combination, within 2 cm of the central axis of the stylet.

In an embodiment, wherein handle and stylet provide a substantially clear sight line of at least 270 degrees along a dorsal line of the handle and stylet combination, within 1 cm of the central axis of the stylet.

In an embodiment, a intubation tool for assisting in placement of an endotracheal tube, the endotracheal intubation tool is included having a) a stylet having a medial location and a distal end, the stylet: i.) configured for receiving an endotracheal tube, and ii) having an articulating distal tip, the articulating tip end being configured to articulate between more than one shape, and b) a handle secured to the medial location of the stylet, wherein actuation is provided by a cable.

In an embodiment, the cable radial location optimized to facilitate articulation and minimize pull force.

In an embodiment, the handle is removable from the stylet.

In an embodiment, the handle is removable from the medial location of the stylet to allow placement of an endotracheal tube in place.

In an embodiment, the stylet articulates in a plane.

In an embodiment, the stylet includes an internal mechanism for articulating the distal end.

In an embodiment, where the stylet further includes a flexible intermediate portion.

In an embodiment, the stylet includes an internal cable connected to the distal end, and wherein pulling on the cable from the medial location results in articulating the distal end of the stylet.

In an embodiment, further can include an actuator.

In an embodiment, the actuator includes a tube surrounding a portion of the medial location of the stylet.

In an embodiment, a method for placement of an endotracheal tube, the method is included, the method a) providing an intubation tool for assisting in placement of an endotracheal tube, the intubation tool is included, the method i) a stylet having a medial location and a distal end, the stylet configured for receiving an endotracheal tube, and having an articulating distal end, the articulating distal end being configured to articulate between more than one shape, and ii) a handle secured to the medial location of the stylet, b) inserting the distal end of the stylet of the endotracheal intubation tool into the trachea of a person, including articulating the distal end of the stylet during insertion to aid in passage through the larynx, c) removing the handle from the medial location of the stylet, d) inserting an endotracheal tube over the medial location of the stylet and down toward the distal end of the stylet, and e) removing the stylet from the patient's trachea while keeping the endotracheal tube in place, wherein the actuation can be performed with a single hand.

In an embodiment, the endotracheal intubation tool for assisting in placement of an endotracheal tube is removable from the medial location of the stylet.

In an embodiment, wherein actuation occurs push/pull action on the tip by allowing three finger, 360 degree contact of circular trigger.

In an embodiment, the endotracheal intubation stylet includes an internal mechanism for articulating the distal end.

In an embodiment, the stylet further includes a flexible intermediate portion.

In an embodiment, the stylet includes an internal cable connected to the distal end, and wherein pulling on the cable from the medial location results in articulating the distal end of the stylet.

In an embodiment, further can include an actuator.

In an embodiment, the actuator includes a tube surrounding a portion of the medial location of the stylet.

In an embodiment, a intubation tool for assisting in placement of an endotracheal tube, the intubation tool is included having a) a stylet having a proximal end, a distal end, and a medial location, the stylet: i) configured for receiving an endotracheal tube, and ii) having an articulating distal tip, the articulating distal tip being configured to articulate between more than one shape, and b) a removable handle secured to a medial location on of the stylet, wherein the stylet has a substantially uniform outer diameter.

In an embodiment, the minimum non-tip diameter of stylet is within 70 percent of the maximum non-tip diameter of the stylet.

In an embodiment, the minimum non-tip diameter of stylet is within 80 percent of the maximum non-tip diameter of the stylet.

In an embodiment, the minimum non-tip diameter of stylet is within 90 percent of the maximum non-tip diameter of the stylet.

In an embodiment, the stylet includes a keyed surface for securing a handle.

In an embodiment, a intubation tool for assisting in placement of an endotracheal tube, the intubation tool is included having a) a stylet having a proximal end, a distal end, and a medial location, the stylet: i) configured for receiving an endotracheal tube, and ii) having an articulating distal tip, the articulating distal tip being configured to articulate between more than one shape, and b) a removable handle secured to a medial location on of the stylet wherein the stylet includes a flexible cable to articulate the distal tip.

In an embodiment, the distal end of the stylet is in a neutral position when no articulating force is applied.

In an embodiment, the force to articulate the distal end is less than 2 pounds.

In an embodiment, the force to articulate the distal end is less than 4 pounds.

In an embodiment, the force to articulate the distal end from 60 to 90 degrees is within 150 percent of the force to articulate the distal end from 0 to 30 degrees.

In an embodiment, the force to articulate the distal end from 80 to 90 degrees is within 150 percent of the force to articulate the distal end from 0 to 10 degrees.

In an embodiment, a intubation tool for assisting in placement of an endotracheal tube, the intubation tool is included having a) a stylet having a proximal end, a distal end, and a medial location, the stylet: i) configured for receiving an endotracheal tube, and ii) having an articulating distal tip, the articulating distal tip being configured to articulate between more than one shape, and the articulating distal tip includes a plurality of free vertebrate: and b) a removable handle secured to a medial location on of the stylet.

In an embodiment, the vertebrate have a substantially flat peak

In an embodiment, the vertebrate have a substantially flat valley

In an embodiment, the vertebrate interface angle is from 10 to 20 degrees.

In an embodiment, wherein handle and stylet provide a substantially clear sight line of at least 90 degrees along a dorsal line of the handle and stylet combination, within 1 cm of the central axis of the stylet.

In an embodiment, wherein handle and stylet provide a substantially clear sight-line along the length of the stylet when the stylet is in a non-articulated mode.

In an embodiment, the articulating distal tip can be articulated with a force of 4 to 9 Newton.

In an embodiment, the handle of the tool includes a grip extension oriented at 90 degrees to the plane of movement of the distal end of the stylus.

In an embodiment, the distal end of the stylet is in a neutral position when no articulating force is applied.

In an embodiment, a intubation tool for assisting in placement of an endotracheal tube, the intubation tool is included having a) a stylet having a proximal end, a distal end, and a medial location, the stylet: i) configured for receiving an endotracheal tube, and ii) having an articulating distal tip, the articulating distal tip being configured to articulate between more than one shape, and b) a removable handle secured to a medial location on of the stylet, wherein the articulating tip is not rigid.

In an embodiment, the force to articulate the distal end from 60 to 90 degrees is within 150 percent of the force to articulate the distal end from 0 to 30 degrees.

In an embodiment, wherein handle and stylet provide a substantially clear sight line of at least 90 degrees along a dorsal line of the handle and stylet combination, within 1 cm of the central axis of the stylet.

In an embodiment, the articulating distal tip can be articulated with a force of 4 to 9 Newton.

In an embodiment, wherein handle and stylet provide a substantially clear sight-line along the length of the stylet when the stylet is in a non-articulated mode.

In an embodiment, the handle of the tool includes a grip extension oriented at 90 degrees to the plane of movement of the distal end of the stylus.

In an embodiment, a intubation tool for assisting in placement of an endotracheal tube, the intubation tool is included having a) a stylet having a proximal end, a distal end, and a medial location, the stylet: i) configured for receiving an endotracheal tube, and ii) having an articulating distal tip, the articulating distal tip being configured to articulate between more than one shape, and b) a removable handle secured to a medial location on of the stylet, wherein the articulating tip transmits tactile feedback. In an embodiment, the force to articulate the distal end is less than 2 pounds.

In an embodiment, the stylet includes an internal cable connected to the distal end, and wherein pulling on the cable from the medial location results in articulating the distal end of the stylet.

In an embodiment, wherein handle and stylet provide a substantially clear sight-line along the length of the stylet when the stylet is in a non-articulated mode.

In an embodiment, wherein handle and stylet provide a substantially clear sight line of at least 90 degrees along a dorsal line of the handle and stylet combination, within 1 cm of the central axis of the stylet.

In an embodiment, the force to articulate the distal end from 60 to 90 degrees is within 150 percent of the force to articulate the distal end from 0 to 30 degrees.

In an embodiment, the distal end of the stylet is in a neutral position when no articulating force is applied.

In an embodiment, a intubation tool for assisting in placement of an endotracheal tube, the intubation tool is included having a stylet having a proximal end, a distal end, and a medial location, the stylet: i) configured for receiving an endotracheal tube, ii) having an articulating distal tip, the articulating distal tip being configured to articulate between more than one shape, and iii) a slidable actuator articulating the distal tip, the slidable actuator being engageable around at least 180 degree of the central axis of the stylet.

In an embodiment, the slidable actuator is engageable around at least 270 of the central axis of the stylet..

In an embodiment, the slidable actuator is engageable around at 360 of the central axis of the stylet.

In an embodiment, a intubation tool for assisting in placement of an endotracheal tube, the intubation tool is included having a) a stylet having a proximal end, a distal end, and a medial location, the stylet including a bi-lumen extrusion, the stylet: i) configured for receiving an endotracheal tube, and ii) having an articulating distal tip, the articulating distal tip being configured to articulate between more than one shape, and b) a removable handle secured to a medial location on of the stylet.

In an embodiment, the force to articulate the distal end from 60 to 90 degrees is within 150 percent of the force to articulate the distal end from 0 to 30 degrees.

In an embodiment, wherein handle and stylet provide a substantially clear sight-line along the length of the stylet when the stylet is in a non-articulated mode.

In an embodiment, the articulating distal tip can be articulated with a force of 4 to 9 Newton.

In an embodiment, the handle of the tool includes a grip extension oriented at 90 degrees to the plane of movement of the distal end of the stylus.

In an embodiment, wherein handle and stylet provide a substantially clear sight line of at least 90 degrees along a dorsal line of the handle and stylet combination, within 1 cm of the central axis of the stylet.

In an embodiment, the distal end of the stylet is in a neutral position when no articulating force is applied.

In an embodiment, a method for placement of an endotracheal tube, the method is included, the method a) providing an intubation tool for assisting in placement of an endotracheal tube, the intubation tool is included, the method i) a stylet having a proximal end, distal end, and intermediate medial portion, the stylet configured for receiving an endotracheal tube, and having an articulating distal tip, the articulating distal tip being configured to articulate between more than one shape using one to three fingers, and ii) a handle secured to a medial position on the stylet, b) inserting the distal end of the stylet of the intubation tool into the trachea of a person, including articulating the distal end of the stylet during insertion to aid in passage through the larynx, c) removing the handle from the medial portion of the stylet, d) placing an endotracheal tube over the medial location of the stylet and down toward the distal end of the stylet and into the trachea of the person, and e) removing the stylet from the patient's trachea while keeping the endotracheal tube in place.

**In** an embodiment, the force to articulate the distal end from 60 to 90 degrees is within 150 percent of the force to articulate the distal end from 0 to 30 degrees.

**In** an embodiment, the stylet includes an internal cable connected to the distal end, and wherein pulling on the cable from the medial location results in articulating the distal end of the stylet.

**In** an embodiment, the articulating distal tip can be articulated with a force of 4 to 9 Newton.

**In** an embodiment, the handle of the tool includes a grip extension oriented at 90 degrees to the plane of movement of the distal end of the stylus.

**In** an embodiment, a intubation tool for assisting in placement of an endotracheal tube, the endotracheal intubation tool is included having a) a stylet having a medial location and a distal end, the stylet: i.) configured for receiving an endotracheal tube, and ii) having an articulating distal tip, the articulating tip end being configured to articulate between more than one shape, and b) a handle secured to the medial location of the stylet, wherein actuation is provided by a cable.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the scope herein.

## Claims

1. An intubation tool for assisting in placement of an endotracheal tube, the intubation tool comprising:
a) a stylet having a proximal end, a distal end, and a medial location, the stylet:
i) configured for receiving an endotracheal tube, and
ii) having an articulating distal tip, the articulating distal tip being configured to articulate between more than one shape
a transition segment located intermediate the less stiff distal end of the stylet and a more stiff proximal portion of the stylet, the transition segment having a distal portion and proximal portion, the transition segment having two parallel openings along its length, a medial portion with a diameter greater than a distal portion and proximal portion of the transition segment, with the distal portion and proximal portion each having an outer diameter less than the outer diameter of each of the distal end of the stylet and the less flexible proximal portion of the style and fitting within the distal end and proximal portion of the stylet such that the distal end and proximal portion of the stylet and the medial portion of the transition segment have substantially the same diameter dimeter; and
an upper sheath portion and a separate lower sheath portion, the upper sheath portion and the lower sheath portion comprising pathways for an upper control wire and a lower control wire, the upper control wire and lower control wire located in separate openings in the transition segment; wherein the upper and lower sheath portions are proximal to the transition segment and terminate within the transition segment; and
b) a handle removably secured to the proximal end of the stylet;
wherein a single slidable steering control base completely surrounds the stylet and is slidable along the stylet to articulate the distal end of the stylet; and wherein the steering control base comprises a slidable tube surrounding a portion of the proximal end of the stylet configured to move forward and backward to articulate the distal end of the stylet; the steering control base having a diameter narrow enough to pass through an endotracheal tube.

2. The intubation tool for assisting in placement of an endotracheal tube of claim **1,** wherein articulation of the distal tip of the stylet can be accomplished with a single hand that holds the removable handle and articulates the distal end.

3. The intubation tool for assisting in placement of an endotracheal tube of any of claims 1-2, wherein articulation of the distal tip of the stylet can be accomplished with a two fingers manipulating a steering control mounted on the stylet, the steering control sliding along the stylet.

4. The intubation tool for assisting in placement of an endotracheal tube of any of claims 1-3, wherein translation movement of a steering control by a distance D results in bending articulation of the distal tip by a distance of at least 150 percent of D.

5. The intubation tool for assisting in placement of an endotracheal tube of any of claims 1-4, wherein translation movement of the steering control in a first direction results in bending articulation of the articulating distal tip in a first direction; and
translational movement of the steering control in an opposite direction results in bending articulation of the articulating distal tip in a direction opposite the first direction.

6. The intubation tool for assisting in placement of an endotracheal tube of any of claims 1-5, wherein the stylet comprises bilumen tubing.

7. The intubation tool for assisting in placement of an endotracheal tube of any of claims 1-6, wherein upon removal of the handle from the medial portion of the stylet, the medial portion of the stylet has a diameter no greater than 120 percent of the remainder of the stylet.

8. The intubation tool for assisting in placement of an endotracheal tube of any of claims 1-7, wherein the widest portion of the stylet after removal of the handle is less than the diameter of the interior of the lumen of an endotracheal tube to be installed on the stylet.

9. The intubation tool for assisting in placement of an endotracheal tube of any of claims 1-8, wherein handle and stylet provide a clear sight line of angle α of 20 degrees along a dorsal elevation LD of 1 cm.

10. The intubation tool for assisting in placement of an endotracheal tube of any of claims 1-9, wherein the handle and stylet provide a clear sight line of angle α of 90 degrees along a dorsal elevation LD of 1 cm.

11. The intubation tool for assisting in placement of an endotracheal tube of any of claims 1-10, wherein the stylet has a length at least 2.0 times the length of an endotracheal tube to be installed on the stylet.

12. The intubation tool for assisting in placement of an endotracheal tube of any of claims 1-11, wherein handle and stylet provide a substantially clear sight-line along the length of the stylet when the stylet is in a non-articulated mode.

13. The intubation tool for assisting in placement of an endotracheal tube of any of claims 1-12, wherein handle and stylet provide a substantially clear sight line of at least 180 degrees along the dorsal line of the handle and stylet combination.

14. The intubation tool for assisting in placement of an endotracheal tube of any of claims 1-13, wherein handle and stylet provide a substantially clear sight line of at least 270 degrees along a dorsal line of the handle and stylet combination, within 1 cm of the central axis of the stylet.

15. The intubation tool for assisting in placement of an endotracheal tube of any of claims 1-14, wherein the handle is removable from the medial location of the stylet to allow placement of an endotracheal tube in place.

## Patentansprüche

1. Intubationswerkzeug zum Unterstützen bei der Platzierung eines Endotrachealtubus, das Intubationswerkzeug umfassend:
a) ein Stilett, das ein proximales Ende, ein distales Ende und eine mittlere Stelle aufweist, wobei das Stilett:
i) konfiguriert ist, um einen Endotrachealtubus zu empfangen, und
ii) eine gelenkige distale Spitze aufweist, wobei die gelenkige distale Spitze konfiguriert ist, um sich zwischen mehr als einer Form zu bewegen
ein Übergangssegment, das sich zwischen dem weniger steifen distalen Ende des Stilettes und einem steiferen proximalen Abschnitt des Stilettes befindet, wobei das Übergangssegment einen distalen Abschnitt und einen proximalen Abschnitt aufweist, wobei das Übergangssegment zwei parallele Öffnungen entlang seiner Länge aufweist, einen medialen Abschnitt mit einem Durchmesser, der größer ist als der distale Abschnitt und der proximale Abschnitt des Übergangssegmentes, wobei der distale Abschnitt und der proximale Abschnitt jeweils einen Außendurchmesser aufweisen, der kleiner ist als der Außendurchmesser des distalen Endes des Stilettes und des weniger flexiblen proximalen Abschnitts des Stilettes und so in das distale Ende und den proximalen Abschnitt des Stilettes passen, dass das distale Ende und der proximale Abschnitt des Stilettes und der mittlere Abschnitt des Übergangssegments im Wesentlichen denselben Durchmesser aufweisen; und
einen oberen Hüllenabschnitt und einen separaten unteren Hüllenabschnitt, wobei der obere Hüllenabschnitt und der untere Hüllenabschnitt Pfade für einen oberen Steuerdraht und einen unteren Steuerdraht umfassen, wobei der obere Steuerdraht und der untere Steuerdraht in separaten Öffnungen in dem Übergangssegment angeordnet sind; wobei der obere und der untere Hüllenabschnitt proximal zu dem Übergangssegment liegen und innerhalb des Übergangssegments enden; und
b) einen Griff, der abnehmbar am proximalen Ende des Stiletts befestigt ist;
wobei eine einzelne verschiebbare Steuerbasis das Stilett vollständig umgibt und entlang des Stiletts verschiebbar ist, um das distale Ende des Stiletts zu artikulieren; und wobei die Steuerbasis ein verschiebbares Rohr umfasst, das einen Abschnitt des proximalen Endes des Stiletts umgibt und konfiguriert ist, um sich vorwärts und rückwärts zu bewegen, um das distale Ende des Stiletts zu artikulieren; wobei die Steuerbasis einen Durchmesser aufweist, der eng genug ist, um durch einen Endotrachealtubus zu passen.

2. Intubationswerkzeug zum Unterstützen der Platzierung eines Endotrachealtubus nach Anspruch 1, wobei die Artikulation der distalen Spitze des Stiletts mit einer einzigen Hand durchgeführt werden kann, die den abnehmbaren Griff hält und das distale Ende artikuliert.

3. Intubationswerkzeug zum Unterstützen der Platzierung eines Endotrachealtubus nach einem der Ansprüche 1 bis 2, wobei die Artikulation der distalen Spitze des Stiletts mit zwei Fingern erreicht werden kann, die eine an dem Stilett befestigte Lenksteuerung bedienen, wobei die Lenksteuerung entlang des Stiletts gleitet.

4. Intubationswerkzeug zum Unterstützen der Platzierung eines Endotrachealtubus nach einem der Ansprüche 1 bis 3, wobei eine Translationsbewegung einer Lenksteuerung um eine Strecke D zu einer Biegung der distalen Spitze um eine Strecke von mindestens 150 Prozent von D führt.

5. Intubationswerkzeug zum Unterstützen der Platzierung eines Endotrachealtubus nach einem der Ansprüche 1 bis 4, wobei eine Translationsbewegung der Lenksteuerung in eine erste Richtung zu einer Biegung der gelenkigen distalen Spitze in eine erste Richtung führt; und
eine Translationsbewegung der Lenksteuerung in eine entgegengesetzte Richtung zu einer Beugung der gelenkigen distalen Spitze in eine der ersten Richtung entgegengesetzte Richtung führt.

6. Intubationswerkzeug zum Unterstützen der Platzierung eines Endotrachealtubus nach einem der Ansprüche 1 bis 5, wobei das Stilett ein Bilumenrohr umfasst.

7. Intubationswerkzeug zum Unterstützen der Platzierung eines Endotrachealtubus nach einem der Ansprüche 1 bis 6, wobei beim Entfernen des Griffs vom mittleren Abschnitt des Stilettes der mittlere Abschnitt des Stilettes einen Durchmesser von nicht mehr als 120 Prozent des Restes des Stilettes aufweist.

8. Intubationswerkzeug zum Unterstützen der Platzierung eines Endotrachealtubus nach einem der Ansprüche 1 bis 7, wobei der breiteste Abschnitt des Stiletts nach Entfernen des Griffs kleiner ist als der Durchmesser des Inneren des Lumens eines auf dem Stiletts zu installierenden Endotrachealtubus.

9. Intubationswerkzeug zum Unterstützen der Platzierung eines Endotrachealtubus nach einem der Ansprüche 1 bis 8, wobei Griff und Stilett eine klare Sichtlinie mit einem Winkel α von 20 Grad entlang einer dorsalen Elevation LD von 1 cm bereitstellen.

10. Intubationswerkzeug zum Unterstützen der Platzierung eines Endotrachealtubus nach einem der Ansprüche 1 bis 9, wobei der Griff und Stilett eine klare Sichtlinie mit einem Winkel α von 90 Grad entlang einer dorsalen Elevation LD von 1 cm bereitstellen.

11. Intubationswerkzeug zum Unterstützen der Platzierung eines Endotrachealtubus nach einem der Ansprüche 1 bis 10, wobei das Stilett mindestens die 2,0-fache Länge eines auf dem Stilett zu installierenden Endotrachealtubus aufweist.

12. Intubationswerkzeug zum Unterstützen der Platzierung eines Endotrachealtubus nach einem der Ansprüche 1 bis 11, wobei Griff und Stilett eine im Wesentlichen freie Sichtlinie entlang der Länge des Stilettes bereitstellen, wenn sich das Stilett in einem nicht artikulierten Modus befindet.

13. Intubationswerkzeug zum Unterstützen der Platzierung eines Endotrachealtubus nach einem der Ansprüche 1 bis 12, wobei Griff und Stilett eine im Wesentlichen freie Sichtlinie von mindestens 180 Grad entlang der dorsalen Linie der Griff-Stilett-Kombination bereitstellen.

14. Intubationswerkzeug zum Unterstützen der Platzierung eines Endotrachealtubus nach einem der Ansprüche 1 bis 13, wobei Griff und Stilett eine im Wesentlichen freie Sichtlinie von mindestens 270 Grad entlang einer dorsalen Linie der Griff-Stilett-Kombination bereitstellen, innerhalb von 1 cm von der Mittelachse des Stilettes.

15. Intubationswerkzeug zum Unterstützen der Platzierung eines Endotrachealtubus nach einem der Ansprüche 1 bis 14, wobei der Griff von der mittleren Stelle des Stiletts abnehmbar ist, um die Platzierung eines Endotrachealtubus an Ort und Stelle zu ermöglichen.

## Revendications

1. Outil d'intubation destiné à faciliter la mise en place d'une sonde endotrachéale, l'outil d'intubation comprenant :
a) un stylet présentant une extrémité proximale, une extrémité distale et un emplacement médian, le stylet :
i) étant conçu pour recevoir une sonde endotrachéale, et
ii) présentant une pointe distale articulée, la pointe distale articulée étant conçue pour s'articuler entre plusieurs formes
un segment de transition situé entre l'extrémité distale moins rigide du stylet et une partie proximale plus rigide du stylet, le segment de transition présentant une partie distale et une partie proximale, le segment de transition présentant deux ouvertures parallèles sur sa longueur, une partie médiane dont le diamètre est supérieur à celui de la partie distale et de la partie proximale du segment de transition, la partie distale et la partie proximale présentant chacune un diamètre extérieur inférieur au diamètre extérieur de chacune de l'extrémité distale du stylet et de la partie proximale moins rigide du stylet et s'adaptant à l'intérieur de l'extrémité distale et de la partie proximale du stylet de sorte que l'extrémité distale et la partie proximale du stylet et la partie médiane du segment de transition aient sensiblement le même diamètre ; et
une partie supérieure de gaine et une partie inférieure de gaine séparée, la partie supérieure de gaine et la partie inférieure de gaine comprenant des chemins pour un fil de commande supérieur et un fil de commande inférieur, le fil de commande supérieur et le fil de commande inférieur étant situés dans des ouvertures séparées dans le segment de transition ; dans lequel les parties supérieure et inférieure de gaine sont proches du segment de transition et se terminent à l'intérieur du segment de transition ; et
b) une poignée fixée de manière amovible à l'extrémité proximale du stylet ;
dans lequel une seule base de commande de direction coulissante entoure complètement le stylet et peut coulisser le long du stylet pour articuler l'extrémité distale du stylet ; et dans lequel la base de commande de direction comprend un tube coulissant entourant une partie de l'extrémité proximale du stylet conçu pour se déplacer d'avant en arrière afin d'articuler l'extrémité distale du stylet ; la base de commande de direction présentant un diamètre suffisamment étroit pour passer à travers une sonde endotrachéale.

2. Outil d'intubation destiné à faciliter la mise en place d'une sonde endotrachéale selon la revendication 1, dans lequel l'articulation de la pointe distale du stylet peut être réalisée d'une seule main qui tient la poignée amovible et articule l'extrémité distale.

3. Outil d'intubation destiné à faciliter la mise en place d'une sonde endotrachéale selon l'une quelconque des revendications 1 et 2, dans lequel l'articulation de la pointe distale du stylet peut être réalisée avec deux doigts manipulant une commande de direction montée sur le stylet, la commande de direction coulissant le long du stylet.

4. Outil d'intubation destiné à faciliter la mise en place d'une sonde endotrachéale selon l'une quelconque des revendications 1 à 3, dans lequel le mouvement de translation d'une commande de direction d'une distance D entraîne la flexion de l'articulation de la pointe distale d'une distance d'au moins 150 % de D.

5. Outil d'intubation destiné à faciliter la mise en place d'une sonde endotrachéale selon l'une quelconque des revendications 1 à 4, dans lequel un mouvement de translation de la commande de direction dans une première direction entraîne la flexion de l'articulation de la pointe distale articulée dans une première direction ; et
un mouvement de translation de la commande de direction dans une direction opposée entraîne la flexion de l'articulation de la pointe distale articulée dans une direction opposée à la première direction.

6. Outil d'intubation destiné à faciliter la mise en place d'une sonde endotrachéale selon l'une quelconque des revendications 1 à 5, dans lequel le stylet comprend un tube à double lumière.

7. Outil d'intubation destiné à faciliter la mise en place d'une sonde endotrachéale selon l'une quelconque des revendications 1 à 6, dans lequel, lors du retrait de la poignée de la partie médiane du stylet, la partie médiane du stylet présente un diamètre qui n'est pas supérieur à 120 % du reste du stylet.

8. Outil d'intubation destiné à faciliter la mise en place d'une sonde endotrachéale selon l'une quelconque des revendications 1 à 7, dans lequel la partie la plus large du stylet après retrait de la poignée est inférieure au diamètre intérieur de la lumière d'une sonde endotrachéale à installer sur le stylet.

9. Outil d'intubation destiné à faciliter la mise en place d'une sonde endotrachéale selon l'une quelconque des revendications 1 à 8, dans lequel la poignée et le stylet offrent une ligne de vue dégagée d'angle α de 20 degrés le long d'une élévation dorsale LD de 1 cm.

10. Outil d'intubation destiné à faciliter la mise en place d'une sonde endotrachéale selon l'une quelconque des revendications 1 à 9, dans lequel la poignée et le stylet offrent une ligne de vue dégagée d'angle α de 90 degrés le long d'une élévation dorsale LD de 1 cm.

11. Outil d'intubation destiné à faciliter la mise en place d'une sonde endotrachéale selon l'une quelconque des revendications 1 à 10, dans lequel le stylet présente une longueur au moins 2,0 fois la longueur d'une sonde endotrachéale à installer sur le stylet.

12. Outil d'intubation destiné à faciliter la mise en place d'une sonde endotrachéale selon l'une quelconque des revendications 1 à 11, dans lequel la poignée et le stylet offrent une ligne de vue sensiblement dégagée sur toute la longueur du stylet lorsque le stylet est en mode non articulé.

13. Outil d'intubation destiné à faciliter la mise en place d'une sonde endotrachéale selon l'une quelconque des revendications 1 à 12, dans lequel la poignée et le stylet offrent une ligne de vue sensiblement dégagée d'au moins 180 degrés le long de la ligne dorsale de la combinaison poignée et stylet.

14. Outil d'intubation destiné à faciliter la mise en place d'une sonde endotrachéale selon l'une quelconque des revendications 1 à 13, dans lequel la poignée et le stylet offrent une ligne de vue sensiblement dégagée d'au moins 270 degrés le long d'une ligne dorsale de la combinaison poignée et stylet, à moins de 1 cm de l'axe central du stylet.

15. Outil d'intubation destiné à faciliter la mise en place d'une sonde endotrachéale selon l'une quelconque des revendications 1 à 14, dans lequel la poignée est amovible de l'emplacement médian du stylet pour permettre la mise en place d'une sonde endotrachéale.
